(19) Europäisches Patentamt / European Patent Office / Office européen des brevets

(11) **EP 3 556 767 A1**

(12) **EUROPEAN PATENT APPLICATION**

(43) Date of publication:
**23.10.2019 Bulletin 2019/43**

(51) Int Cl.:
***C07K 7/08*** *(2006.01)*

(21) Application number: **18382261.8**

(22) Date of filing: **18.04.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Universidade De Santiago De Compostela**
**15782 Santiago de Compostela - A Coruña (ES)**

(72) Inventors:
• **MONTENEGRO GARCÍA, Javier**
 **15782 Santiago de Compostela (A CORUÑA) (ES)**
• **JUANES CARRASCO, María Luisa**
 **15782 Santiago de Compostela (A CORUÑA) (ES)**
• **LOSTALÉ-SEIJO, Irene**
 **15782 Santiago de Compostela (A CORUÑA) (ES)**
• **GALLEGO GÓMEZ, Iván**
 **15782 Santiago de Compostela (A CORUÑA) (ES)**
• **REINA-MARTÍN, José Juan**
 **15782 Santiago de Compostela (A CORUÑA) (ES)**
• **PAZO PASCUAL, Marta**
 **15782 Santiago de Compostela (A CORUÑA) (ES)**
• **SALLUCE, Giulia**
 **15782 Santiago de Compostela (A CORUÑA) (ES)**

(74) Representative: **Balder IP Law, S.L.**
 **Paseo de la Castellana 93**
 **5ª planta**
 **28046 Madrid (ES)**

Remarks:
A request for correction of the drawings has been filed pursuant to Rule 139 EPC. A decision on the request will be taken during the proceedings before the Examining Division (Guidelines for Examination in the EPO, A-V, 3.).

(54) **CELL PENETRATING PEPTIDES**

(57) The present invention relates to a cell penetrating peptide with or without a ligand comprising a scaffold of at least two hydrophobic blocks and at least two cationic blocks, each cationic blocks alternating with one hydrophobic block; wherein the total number of amino acids is comprised between 5 and 40, and wherein each of said hydrophobic blocks individually considered consist of one or more hydrophobic amino acids, wherein at least one of said hydrophobic amino acids is selected from the group consisting of leucine, alanine and isoleucine; wherein said at least two hydrophobic blocks comprise at least a leucine amino acid; and each of said cationic blocks individually considered comprise at least one cationic amino acid having a side chain comprising a nitrogen atom, preferably one which is protonated under physiological conditions.

SEQUENCE LISTING

<110> Universidade de Santiago de Compostela

<120> cell penetrating peptides

<130> P180832EP

<160> 16

<170> PatentIn version 3.5

<210> 1
<211> 16
<212> PRT
<213> N/A


<220>
<221> MOD_RES
<222> (1)..(1)
<223> 5-carboxytetramethyl rhodamine (TAMRA) in position 1 (Ala)

<400> 1

Ala Arg Ala Ala Ala Ala Ala Ala Arg Ala Ala Ala Ala Ala Arg
1               5                   10                  15


<210> 2
<211> 16
<212> PRT
<213> N/A


<220>
<221> MOD_RES
<222> (1)..(1)
<223> 55-carboxytetramethyl rhodamine (TAMRA) in position 1 (Ala)

<400> 2

Ala Arg Ala Leu Ala Ala Leu Ala Arg Ala Ala Ala Ala Ala Arg
1               5                   10                  15


<210> 3
<211> 16
<212> PRT
<213> N/A


<220>
<221> MOD_RES
<222> (1)..(1)
<223> 5-carboxytetramethyl rhodamine (TAMRA) in position 1 (Ala)
      through aminohexanoic acid (-(C=O)-(CH2)5-N(H)-)

<400> 3

Ala Arg Ala Ala Leu Leu Ala Ala Arg Ala Ala Leu Ala Ala Ala Arg
1               5                   10                  15

```
<210>    4
<211>    16
<212>    PRT
<213>    N/A


<220>
<221>    MOD_RES
<222>    (1)..(1)
<223>    5-carboxytetramethyl rhodamine (TAMRA) in position 1 (Leu)


<400>    4

Leu Arg Ala Leu Arg Arg Leu Ala Ala Ala Ala Ala Ala Ala Ala Ala
1                5                10                  15


<210>    5
<211>    16
<212>    PRT
<213>    N/A


<220>
<221>    MOD_RES
<222>    (1)..(1)
<223>    5-carboxytetramethyl rhodamine (TAMRA) in position 1 (Leu)
         through aminohexanoic acid (-(C=O)-(CH2)5-N(H)-)


<400>    5

Leu Arg Ala Ala Ala Ala Ala Leu Arg Ala Ala Ala Ala Ala Leu Arg
1                5                10                  15


<210>    6
<211>    16
<212>    PRT
<213>    N/A


<220>
<221>    MOD_RES
<222>    (1)..(1)
<223>    5-carboxytetramethyl rhodamine (TAMRA) in position 1 (Ala)
         through aminohexanoic acid (-(C=O)-(CH2)5-N(H)-)


<400>    6

Ala Arg Ala Leu Ala Ala Leu Ala Arg Ala Leu Ala Ala Ala Ala Arg
1                5                10                  15


<210>    7
<211>    16
<212>    PRT
<213>    N/A


<220>
<221>    MOD_RES
<222>    (1)..(1)
```

<223> 5-carboxytetramethyl rhodamine (TAMRA) in position 1 (Ala)

<400> 7

Ala Arg Ala Leu Ala Ala Leu Ala Arg Ala Leu Ala Ala Ala Ala Arg
1               5                   10                  15


<210> 8
<211> 16
<212> PRT
<213> N/A


<220>
<221> MOD_RES
<222> (1)..(1)
<223> 5-carboxytetramethyl rhodamine (TAMRA) in position 1 (Leu)

<400> 8

Leu Arg Ala Leu Ala Ala Leu Ala Arg Ala Ala Ala Ala Ala Ala Arg
1               5                   10                  15


<210> 9
<211> 16
<212> PRT
<213> N/A


<220>
<221> MOD_RES
<222> (1)..(1)
<223> ACETYLATION

<400> 9

Leu Arg Ala Leu Ala Ala Leu Ala Arg Ala Ala Ala Ala Ala Ala Arg
1               5                   10                  15


<210> 10
<211> 13
<212> PRT
<213> N/A


<220>
<221> MOD_RES
<222> (1)..(1)
<223> ACETYLATION in position 1 (Arg)

<220>
<221> MOD_RES
<222> (2)..(2)
<223> (-(C=O)-(CH2)-O-N=(CH)-(CH2)-alpha-D-Mannose) in position 2 (Lys)

<220>
<221> MOD_RES
<222> (11)..(11)
<223> (-(C=O)-(CH2)-O-N=(CH)-(CH2)-alpha-D-Mannose) in position 11
      (Lys)

<400> 10

Arg Lys Leu Arg Arg Leu Leu Arg Arg Leu Lys Arg Leu
1               5                   10


<210> 11
<211> 13
<212> PRT
<213> N/A


<220>
<221> MOD_RES
<222> (1)..(1)
<223> 5-carboxytetramethyl rhodamine (TAMRA) in position 1 (Arg)


<220>
<221> MOD_RES
<222> (2)..(2)
<223> (-(C=O)-(CH2)-O-N=(CH)-(CH2)-alpha-D-Mannose) in position 2 (Lys)


<220>
<221> MOD_RES
<222> (11)..(11)
<223> (-(C=O)-(CH2)-O-N=(CH)-(CH2)-alpha-D-Mannose) in position 11
      (Lys)


<400> 11

Arg Lys Leu Arg Arg Leu Leu Arg Arg Leu Lys Arg Leu
1               5                   10


<210> 12
<211> 13
<212> PRT
<213> N/A


<220>
<221> MOD_RES
<222> (1)..(1)
<223> ACETYLATION
ACETYLATION in position 1 (Arg)


<220>
<221> MOD_RES
<222> (2)..(2)
<223> -C(=O)-O-(CH)2-(CH)=(CH2) in position 2  (Lys)


<220>
<221> MOD_RES
<222> (11)..(11)
<223> (-(C=O)-(CH2)-O-N=(CH)-(CH2)-alpha-D-Mannose) in position 11
      (Lys)


<400> 12

Arg Lys Leu Arg Arg Leu Leu Arg Arg Leu Lys Arg Leu
1               5                   10

```
<210>   13
<211>   13
<212>   PRT
<213>   N/A


<220>
<221>   MOD_RES
<222>   (1)..(1)
<223>   5-carboxytetramethyl rhodamine (TAMRA) in position 1 (Arg)


<220>
<221>   MOD_RES
<222>   (2)..(2)
<223>   -C(=O)-O-(CH)2-(CH)=(CH2) in position 2 (Lys)


<220>
<221>   MOD_RES
<222>   (11)..(11)
<223>   (-(C=O)-(CH2)-O-N=(CH)-(CH2)-alpha-D-Mannose) in position 11
        (Lys)


<400>   13


Arg Lys Leu Arg Arg Leu Leu Arg Arg Leu Lys Arg Leu
1               5               10


<210>   14
<211>   13
<212>   PRT
<213>   N/A


<220>
<221>   MOD_RES
<222>   (1)..(1)
<223>   ACETYLATION
in position 1 (Arg)


<220>
<221>   MOD_RES
<222>   (2)..(2)
<223>   (-(C=O)-(CH2)4-biotin) in position 2 (Lys)


<220>
<221>   MOD_RES
<222>   (11)..(11)
<223>   (-(C=O)-(CH2)4-biotin) in position 11 (Lys)


<400>   14


Arg Lys Leu Arg Arg Leu Leu Arg Arg Leu Lys Arg Leu
1               5               10


<210>   15
<211>   13
<212>   PRT
<213>   N/A
```

```
<220>
<221>  MOD_RES
<222>  (1)..(1)
<223>  Dextamethasone acid in position 1 (Arg)

<220>
<221>  MOD_RES
<222>  (2)..(2)
<223>  (-(C=O)-(CH2)-O-N=(CH)-(CH2)-alpha-D-Mannose) in position 2 (Lys)

<220>
<221>  MOD_RES
<222>  (11)..(11)
<223>  (-(C=O)-(CH2)-O-N=(CH)-(CH2)-alpha-D-Mannose) in position 11
       (Lys)

<400>  15

Arg Lys Leu Arg Arg Leu Leu Arg Arg Leu Lys Arg Leu
1               5                   10


<210>  16
<211>  13
<212>  PRT
<213>  N/A


<220>
<221>  MOD_RES
<222>  (1)..(1)
<223>  5-carboxytetramethyl rhodamine (TAMRA) in position 1 (Arg)

<220>
<221>  MOD_RES
<222>  (2)..(2)
<223>  (-(C=O)-(CH2)-O-N=(CH)-(CH2)-Glucose) in position 2 (Lys)

<220>
<221>  MOD_RES
<222>  (11)..(11)
<223>  (-(C=O)-(CH2)-O-N=(CH)-(CH2)-Glucose) in position 11 (Lys)

<400>  16

Arg Lys Leu Arg Arg Leu Leu Arg Arg Leu Lys Arg Leu
1               5                   10
```

**Description**

**FIELD OF THE INVENTION**

The present invention relates to cell penetrating peptides, i.e. peptides capable of releasing a molecule of biological interest into the cytosol of the cell. The invention also discloses methods for the preparation of the CPPs and uses thereof.

**BACKGROUND OF THE INVENTION**

The selective transport of proteins across the cell membrane is a central challenge in protein therapy and chemical biology. The emergence of protein therapeutics demands innovative methods for the intracellular delivery of exogenous antibodies, programmable enzymes and transcription factors. Furthermore, expression of proteins by standard plasmid transfection or by mRNA protocols still suffers from significant limitations related with permanent recombination and/or polynucleotide instability. Therefore, as biotechnology evolves, there is a need to develop conceptually new ways to transport and deliver proteins inside cells. Classical approaches for protein transport include liposome encapsulation, viral delivery, covalent capture, electroporation and translational fusion to penetrating tags. Nature-inspired penetrating peptides are among the most commonly employed tags in translational fusion. However, protein fusion requires tedious steps of cloning, bacterial expression and purification. Thus, there is a large body of research in strategies to deliver large molecules, such as proteins, into the cell.

A first strategy seeks the development of supramolecular vectors that can selectively recognize macromolecules through appropriate ligands and transport them across the cell membrane, without further manipulation. Positive results in this direction are of great interest and would represent a conceptual breakthrough towards the design of selective protein delivery vehicles. In this respect, lectins are carbohydrate-specific proteins that participate in numerous biological functions including cellular recognition and communication. Reports on peptide folding for improved glycan presentation and lectin binding can be found scattered throughout the literature (G. L. Simpson, A. H. Gordon, D. M. Lindsay, N. Promsawan, M. P Crump, K. Mulholland, B. R. Hayter, T. Gallagher, J. Am. Chem. Soc. 2006, 128, 10638-10639). Furthermore, lectins are commonly overexpressed during tissue inflammation and cellular metastasis (H. Lis, N. Sharon, Chem. Rev. 1998, 98, 637-674; N. Yamazaki, S. Kojima, N. V. Bovin, S. Andre, S. Gabius, H. J. Gabius, Adv. Drug. Deliv. Rev. 2000, 43, 225-244.). As a consequence, the delivery of exogenous lectins and their conjugates has been explored in the context of cell targeting and cancer therapy (A. Amin, M. L. Oo, T. Senga, N. Suzuki, G. S. Feng, M. Hamaguchi, Cancer Res. 2003, 63, 6334-6339.). Balanced polymeric glycopeptide hybrids for gene delivery and nanocapsules for protein and lectin delivery have been recently developed. However, efforts to equip short penetrating peptides with glycan moieties have met with limited success (L. Dutot, P. Lécorché, F. Burlina, R. Marquant, V. Point, S. Sagan, G. Chassaing, J.-M. Mallet, S. Lavielle, J. Chem. Biol. 2010, 3, 51-65.). Thus, this strategy is focused on vehicles that associate to and transport large molecules of interest.

A second strategy of interest is the provision of molecules that do not contain ligands and thus do not necessarily associate with the molecule of biological interest, but that allow their liberation into the cytosol. In this sense, positively charged molecules and macromolecules with a certain degree of hydrophobicity can interact with anionic membranes and be taken up by cells. Initial evidences of these penetrating capacities were found in sequences of proteins enriched in basic amino acids (P.M. Fischer, Med.Res.Rev., 2007, 27, 755-795; D. Kalafatovic and E. Giralt, Molecules, 2017, 22, 1929-1967). Despite the large progress achieved during the last years, finding CPPs with improved penetrating properties still remains a challenge. Therefore, methodologies for the rational design of penetrating peptide sequences are essential tools for the discovery of the next generation of CPPs with increased efficiency and reduced toxicity. Some elegant strategies building on peptide cyclization, structural fixation and hydrophobic accumulation have been recently studied to rationalize the penetrating capabilities of CPPs. There is however still a need for improved CPPs.

The search of improved CPPs, whether they are short peptide sequences comprising a ligand in order to associate with a molecule of interest and act as transport/carrier, or whether they are short peptide sequences comprising no ligands, has proven a challenging task. Such CPPs must effectively deliver the molecule of interest into the cytosol, and display a low degree of cytotoxicity.

**FIGURES OF THE INVENTION**

Figure 1: Schematic representation of protocol 1 for the synthesis of CPPs. See Example 1.

Figure 2: Schematic representation of protocol 2 for the synthesis of CPPs. See Example 3.

**Figure 3:** Uptake in living HeLa cells of CPPs $P_1$ to $P_8$: uptake quantification by flow cytometry. Cells were incubated with the peptides for 30 minutes at the different concentrations in HKR buffer and trypsinized to remove all membrane bound peptide prior fluorescence quantification by flow cytometry. See example 2.2.

**Figure 4:** Helical wheel representation of CPPs $P_1$ to $P_8$.

**Figure 5:** Uptake quantification by flow cytometry of $P_8$ in three different cell lines: HeLa cells, A549 cells and Sf9 cells. The points were collected at six different concentrations (0, 1, 3, 5, 7, 10 $\mu$M). Cells were incubated with the peptides in DMEM (-) (HeLa and A549) or Sf-900 II (Sf9 cells) for 1 hour prior to trypsinization and flow cytometry quantification. Error bars indicate SD of three replicates. See example 2.2.

**Figure 6:** exemplary structures of ligand-containing CPPs prepared according to example 3.

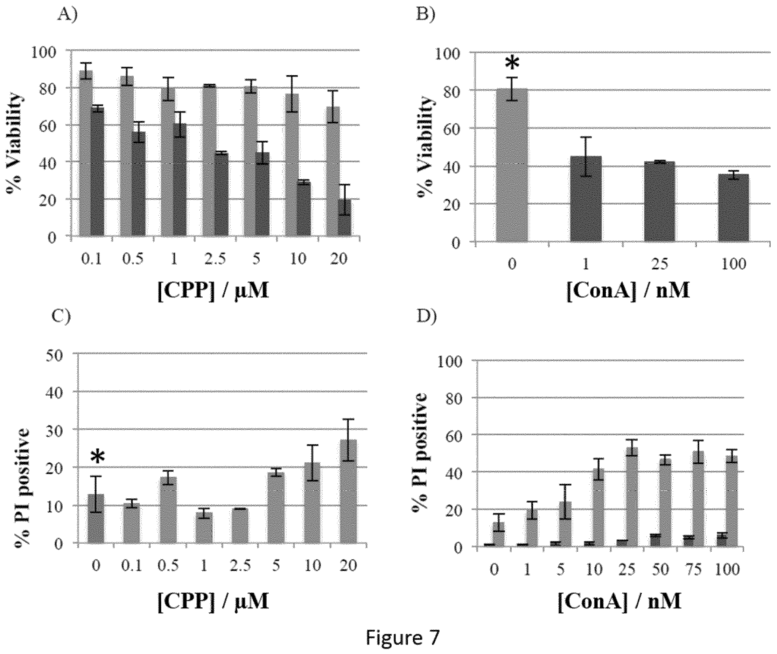

**Figure 7:** Cell viability in HeLa Cells. **A)** MTT assay at different concentrations of AcP(Man)$_2$ (left bar of each pair) and AcP(Acetone)$_2$ (right bar of each pair); **B)** MTT assay at different concentrations of ConA using 5 $\mu$M of AcP(Man)$_2$, the viability for AcP(Man)$_2$ alone at 0 nM of ConA is shown in the first bar (marked with an asterisk); **C)** Propidium iodide staining of dead cells for flow cytometry at different concentrations of AcP(Man)$_2$. The staining of untreated cells (control) is represented in the first bar (marked with an asterisk); **D)** Propidium iodide staining of dead cells for flow cytometry at different concentrations of ConA using 5 $\mu$M of AcP(Man)$_2$ (right bar of each pair). The left bar of each pair represent the cytotoxicity of the ConA alone. See example 4.2.

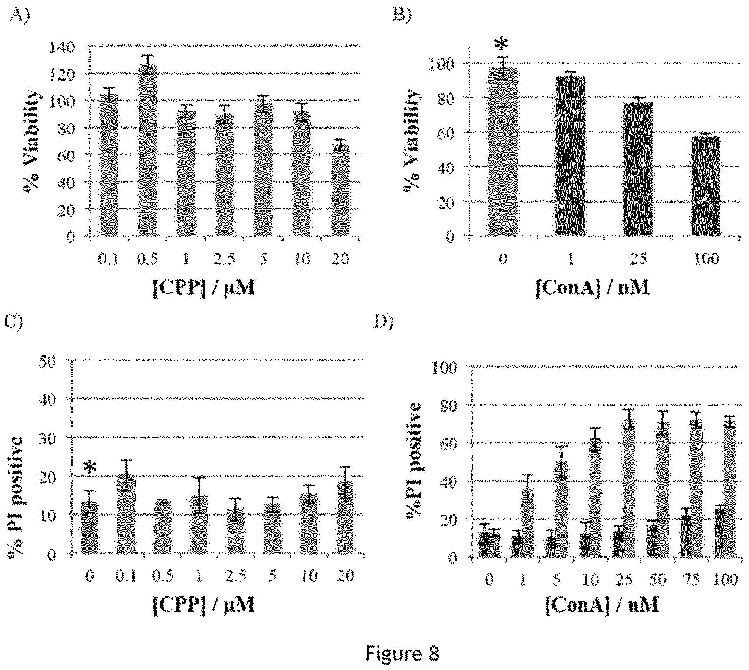

**Figure 8:** Cell viability in HepG2 Cells. **A)** MTT assay at different concentrations of AcP(Man)$_2$; **B)** MTT assay at different concentrations of ConA using 5 $\mu$M of AcP(Man)$_2$, the viability for AcP(Man)$_2$ alone at 0 nM of ConA is shown in the first bar (marked with an asterisk); **C)** Propidium iodide staining of dead cells for flow cytometry at different concentrations of AcP(Man)$_2$. The staining of untreated cells (control) is represented in the first bar (marked with an asterisk); **D)** Propidium iodide staining of dead cells for flow cytometry at different concentrations of ConA using 5 $\mu$M of AcP(Man)$_2$ (right bar of each pair). The left bar of each pair represents the cytotoxicity of the ConA alone. See example 4.2.

**Figure 9:** Release test of saporin in the presence of $P_8$ and $R_8$. Each bar represents the viability in the presence of $P_8$ or $P_8$ + 10 $\mu$g/mL saporin (P8+S) (left panel) or $R_8$ or $R_8$ + 10 $\mu$g/mL saporin (R8 + S) (right panel). See example 2.4.

**Figure 10:** Cre Recombinase intracellular delivery. See example 2.5.

**Figure 11:** Dextran internalization. Dextran (250 $\mu$g/ml) in the presence of 5 $\mu$M of a CPP: (Figure 11A) $P_8$ marked with TAMRA, (Figure 11B) acetylated $P_8$, and (Figure 11C) $R_8$ as comparison. See example 2.3.

**Figure 12:** Figure 12A: endosomal escape captured by confocal microscopy of TmP(Man)$_2$ and TmP(Glu)$_2$ (Tm= TAMRA) at 30 min since the beginning of the incubation and at 60 min (see example 4.3). Figure 12B: medium term toxicity studies of TmP(Man)$_2$ (top) and TmP(Glu)$_2$ (bottom) (Tm= TAMRA). See example 4.2.1, second paragraph. Figure 12C: endosomal escape by glucocorticoid-induced translocation assay (ability of DexP(Man)$_2$ to reach the cytosol). See example 4.4.

# SUMMARY OF THE INVENTION

The inventors have found a group of peptides which provide a surprisingly effective delivery of molecules into the cytosol of the cell. Thus, according to a first aspect, the invention is directed to a cell penetrating peptide (CPP) comprising a scaffold of at least two hydrophobic blocks and at least two cationic blocks, each cationic block alternating with one hydrophobic block;
wherein the total number of amino acids is comprised between 5 and 40, and
wherein

each of said hydrophobic blocks individually considered consist of one or more hydrophobic amino acids, wherein at least one of said hydrophobic amino acids is selected from the group consisting of leucine, alanine and isoleucine; wherein said at least two hydrophobic blocks comprise at least a leucine amino acid; and

each of said cationic blocks individually considered comprise at least one cationic amino acid having a side chain comprising a nitrogen atom, preferably one which is protonated under physiological conditions.

The inventors have discovered that the CPPs of the invention are capable of delivering macromolecules whether or not they are modified to incorporate ligands. If the CPP is modified to include a ligand which associates with a specific molecule of interest, the inventors have observed that the CPP acts as a vehicle or carrier. In this way, the CPP associated with the molecule of interest escapes the endosomes and both are liberated into the cytosol. Thus, the CPP can be modified to incorporate a ligand, for example a monosaccharide or a polysaccharide, attached to the peptide scaffold through an oxime (C=N-O-, e.g. -(C(=O)-(CH$_2$)-O-N=C(H)-CH$_2$- or a hydrazine (C=N-N(H)-). These oxime and hydrazone connections are formed from the corresponding alkoxyamine and hydrazide reacted with the aldehyde. Accordingly, a second aspect of the invention is a complex comprising

A) a cell penetrating peptide comprising a scaffold of at least two hydrophobic blocks and at least two cationic blocks, each cationic block alternating with one hydrophobic block;
wherein the total number of amino acids is comprised between 5 and 40, and
wherein

each of said hydrophobic blocks individually considered consist of one or more hydrophobic amino acids, wherein at least one of said hydrophobic amino acids is selected from the group consisting of leucine, alanine and isoleucine; wherein said at least two hydrophobic blocks comprise at least a leucine amino acid; and

each of said cationic blocks individually considered comprise at least one cationic amino acid having a side chain comprising a nitrogen atom, preferably one which is protonated under physiological conditions;

B) at least one ligand attached to the cell penetrating peptide; and

C) at least one molecule of biological interest associated to the ligand.

On the other hand, if the CPP is used unmodified (does not form a complex with a molecule of interest), it still causes the molecule of interest to be liberated into the cytosol. Without wanting to be bound by theory, it seems that when not forming a complex, the CPPs of the invention act through a different mechanism, although the details have not been entirely studied. It seems that the CPP without any ligands or linkers attached thereto is not liberated into the cytosol. Accordingly, a third aspect of the invention is a composition comprising the cell penetrating peptide of the invention and a molecule of biological interest.

In either case, the molecule of interest escapes the endosome and is liberated into the cytosol. The inventors have been able to fine-tune the properties of the CPPs, and found that, for example, a certain helicity or an alignment of certain amino acids with respect to the axis of the helix improves the liberation of the molecules of interest into the cytosol while maintaining good cytotoxicity levels.

Other aspects of the invention are

- A method for the preparation of the CPPs of the invention comprising the synthesis of a peptidic scaffold by reacting a sequence of aminoacids, and optionally coupling a ligand to said scaffold.

- A pharmaceutical composition comprising the cell penetrating peptide of the invention (first aspect) or a complex as defined above (second aspect) or a composition as defined above (third aspect).

- A cell penetrating peptide of the invention (first aspect) or a complex as defined above (second aspect) or a composition as defined above (third aspect) for use as a medicament.

- A cell penetrating peptide of the invention (first aspect) or a complex as defined above (second aspect) or a composition as defined above (third aspect) for use in delivering into the cytosol a molecule of biological interest.

- A cell penetrating peptide of the invention (first aspect) or a complex as defined above (second aspect) or a composition as defined above (third aspect) for use in the treatment of cancer.

## DESCRIPTION

In the CPP of the invention it is preferred that the cationic amino acids are each individually selected from the group consisting of lysine, arginine and histidine, even more preferably that at least two cationic blocks comprise at least one arginine amino acid. Also, it is preferred that any of the hydrophobic amino acids does not contain a functional group capable of forming a negative or positive charge under physiological conditions, for example, such that the CPP is one wherein at least one hydrophobic amino acid of each hydrophobic block is selected from the group consisting of leucine, alanine and isoleucine; and, preferably, at least one hydrophobic block further comprises at least one hydrophobic amino acid selected from the group consisting of valine, proline, methionine, phenylalanine and tryptophan. Preferably, each hydrophobic block consists of one or more amino acids selected from the group consisting of alanine, leucine and isoleucine. Preferably, the CPP comprises three or more hydrophobic blocks, preferably 4 or more. It is also preferred that the CPP comprises three or more cationic blocks.

In an embodiment of the invention the CPP comprises a reporting moiety.

### Amino acid distribution in CPPs not modified with a ligand

Higher helicities of the CPPs of the invention provide improved uptake in the cell of the CPP. For example, the CPP $P_1$ (comparative) was compared with CPPs $P_2$ to $P_8$ (according to the invention). The percentage of helicity at 40°C for CPPs $P_1$ to $P_8$ are summarized in Table 1:

Table 1

| CPP | % Helicity in Liposomes | % Helicity in water | Amino acid sequence in each CPP (A= alanine; L= leucine; R= Arginine; Tm= TAMRA; Hex= aminohexanoic) |
|---|---|---|---|
| $P_1$ SEQ ID NO: 1 | 0.2 | 9.7 | TmA R $A_6$ R $A_6$ R |
| $P_2$ SEQ ID NO: 2 | 6.5 | 12.4 | TmA R ALAALA R $A_6$ R |
| $P_3$ SEQ ID NO: 3 | 12.7 | 21.7 | TmHexA R AALLAA R AALA$_3$ R |
| $P_4$ SEQ ID NO: 4 | 13.3 | 1.9 | TmL R AL RR LA$_9$ |
| $P_5$ SEQ ID NO: 5 | 29.5 | 19.3 | TmHexL R $A_5$L R $A_5$L R |
| $P_6$ SEQ ID NO: 6 | 29.9 | 35.1 | TmHexA R ALAALA R ALA$_4$ R |
| $P_7$ SEQ ID NO: 7 | 31.6 | 16.1 | TmA R ALAALA R ALA$_4$ R |
| $P_8$ SEQ ID NO: 8 | 49.2 | 12.8 | TmL R ALAALA R $A_6$ R |

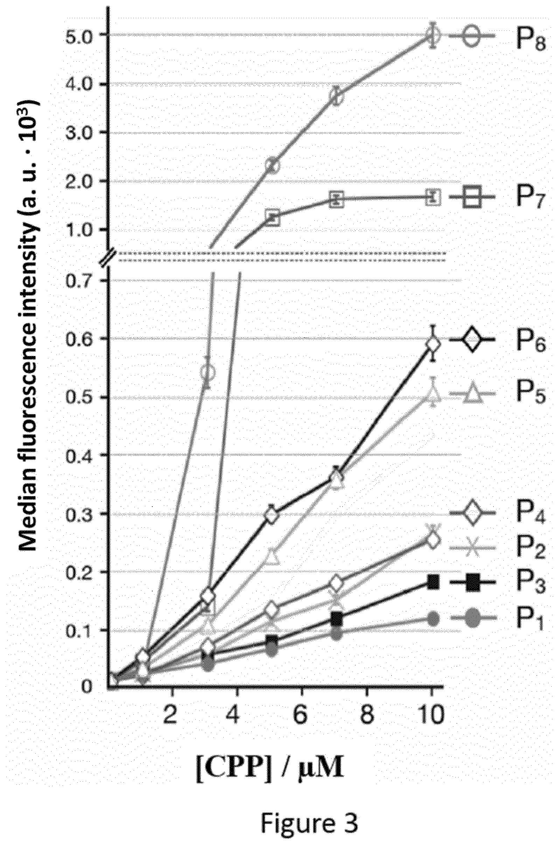

As it can be seen from Table 1 and the uptake results of Figure 3, increases in the helicity in liposomes translate into improved uptake of the CPP. Thus, it is preferred that the CPP of the invention has a percentage of helicity in liposomes greater than 5%, preferably greater than 10%, as measured at 40°C in Milli-Q water in egg yolk phosphatidylcholine liposomes. Preferably, the percentage of helicity in liposomes thereof is greater than 12%, preferably greater than 15%, preferably greater than 20%, 22%, 25%, 26%, 27%, 28%, 29%, 30%, 35%, 40% or than 45%. Thus, for example, the percentage of helicity in liposomes of the CPP of the invention is comprised between 5% and 60%, preferably between 10% and 60%, preferably between 13% and 55%, between 14% and 52%, or between 15% and 50%. The improvement of helicity in water also correlates to an improved uptake of the CPP. Thus, it is preferred that the percentage of helicity of the CPP of the invention in water at 40°C is comprised between 5% and 50%, preferably between 10% and 40%, between 11% and 20%, between 12% and 20%.

Even further, it is clear from the data of Table 1 and Figure 3 that better uptake of the CPP is achieved when the helicity in liposomes is greater than the helicity in water. Accordingly, it is preferable that the percentage of helicity of the CPP in liposomes as measured at 40°C in Milli-Q water in egg yolk phosphatidylcholine liposomes is greater than the percentage of helicity in water at 40°C. Preferably, the difference in the percentage of helicity in liposomes as measured at 40°C in Milli-Q water in egg yolk phosphatidylcholine liposomes and the percentage of helicity in water at 40°C is at least 10, preferably at least 15, more preferably at least 20. CPPs presenting such increased helicity in liposomes with respect to water also provided better cell viability, especially those wherein at least a first cationic amino acid (for example, arginine) of a first cationic block is substantially aligned in the direction perpendicular to the theoretical axis of the helix with at least a second cationic amino acid (for example, arginine) of a second cationic block (see below).

Preferably, at least one of the hydrophobic blocks comprises at least two alanine amino acids, more preferably, at least two of the hydrophobic blocks comprise at least two alanine amino acids. Even more preferably, at least two of the hydrophobic blocks comprise at least two alanine amino acids, at least one of them comprising 3 or more, preferably 4 or more, still more preferably, 5 or more alanine amino acids. Although the scaffold of the CPP of the reference may comprise 5 to 40 amino acids, a total of 15 to 40 aminoacids, preferably 16 to 25, preferably 16 to 21, is preferred.

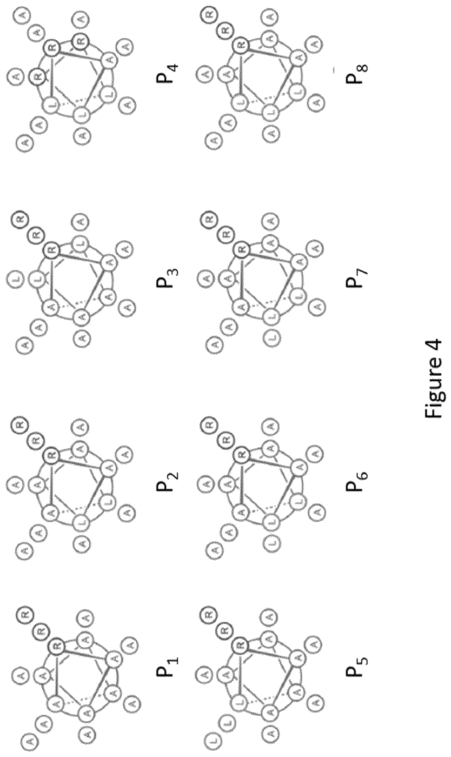

More surprisingly has been the fact that comparative CPP $P_1$, consisting only of alanine and arginine, yielded a very poor helical behaviour and negligible uptake in living HeLa cells (Figure 3), but that the incorporation of two leucine hydrophobic amino acids according to the invention had a clear positive effect on the helicity in liposomes (compare the values of $P_1$ and $P_2$ in Liposomes in Table 1). Further substitution of an extra alanine for a third leucine triggered a higher increase in peptide helicity (compare $P_2$ vs $P_3$) as well as in the cellular uptake of the CPP (see Figure 3). Thus, it is preferred in the present invention that the scaffold of the CPP comprises at least 3 Leucine amino acids. Preferably, at least one of the hydrophobic blocks comprises two leucine amino acids. Further, alignment of the three cationic residues, in this case guanidinium side chains, was clearly beneficial for the helicity in the membrane as it can be observed by comparing $P_4$ and $P_8$ (13% vs 50% helicity in liposomes; note the substantial radial distribution of the arginine amino acids in $P_4$ versus the substantially perpendicular alignment in $P_8$, as shown in Figure 4). It is thus preferable that at least a first cationic amino acid (for example, arginine) of a first cationic block is substantially aligned in the direction perpendicular to the theoretical axis of the helix with at least a second cationic amino acid (for example, arginine) of a second cationic block. This substantially perpendicular alignment improves the uptake of the CPP as shown by the results of Figure 3, wherein $P_5$, $P_6$, $P_7$ and $P_8$ (all having substantially perpendicularly aligned arginine residues (R)) perform better in the uptake test. Preferably, each cationic block comprises at least one arginine which is substantially aligned in the direction perpendicular to the theoretical axis of the helix. It is thus preferred a CPP comprising a first half and a second half separated by a plane containing the theoretical axis of the helix formed by the scaffold, wherein the first half comprises at least two hydrophobic amino acids selected from the group consisting of leucine and isoleucine, and the second half comprises at least two cationic amino acids.

As mentioned above, it is preferred that the CPP comprises 3 leucine aminoacids, preferably, substantially radially distributed around the theoretical axis of the helix (see for example $P_4$ and $P_8$ in Figure 4). This configuration improves uptake: see uptake values in Figure 3 of $P_4$ versus $P_3$, or $P_8$ versus $P_7$.

It is thus preferred that the CPP comprises at least two of the hydrophobic blocks each comprising at least one hydrophobic amino acid selected from the group consisting of leucine and isoleucine, and wherein said leucine or isoleucine amino acids are substantially radially distributed around the direction of the theoretical axis of the helix.

Thus, overall it is preferred a CPP that combines the substantially perpendicular alignment of at least two cationic amino acids, with the substantially radial distribution of the hydrophobic amino acids (preferably leucine or isoleucine), that is a CPP comprising a first half and a second half separated by a plane containing the theoretical axis of the helix formed by the scaffold, wherein the first half comprises at least two hydrophobic amino acids selected from the group consisting of leucine and isoleucine which are substantially radially distributed around the direction of the theoretical axis of the helix, and the second half comprises at least two cationic amino acids substantially aligned in the perpendicular direction of the theoretical axis of the helix. CPPs $P_6$, $P_7$ and $P_8$ fit in this definition.

Preferably, the CPP has a percentage of helicity in liposomes greater than 10%, as measured at 40°C in Milli-Q water in egg yolk phosphatidylcholine liposomes, and comprises a first half and a second half separated by a plane containing the theoretical axis of the helix formed by the scaffold, wherein the first half comprises at least two hydrophobic amino acids selected from the group consisting of leucine and isoleucine substantially radially distributed around the direction of the theoretical axis of the helix, and the second half comprises at least two cationic amino acids substantially aligned in the perpendicular direction of the theoretical axis of the helix.

Also, the CPP can have a percentage of helicity in liposomes greater than 10%, as measured at 40°C in Milli-Q water in egg yolk phosphatidylcholine liposomes, and comprises a first half and a second half when separated by a plane containing the theoretical axis of the helix formed by the scaffold, wherein the first half comprises at least two hydrophobic amino acids selected from the group consisting of leucine and isoleucine substantially radially distributed around the direction of the theoretical axis of the helix, each of said leucine and isoleucine pertaining to different hydrophobic blocks, and the second half comprises at least two cationic amino acids substantially aligned in the perpendicular direction of the theoretical axis of the helix, each of said at least cationic amino acids pertaining to different cationic blocks.

**Amino acid distribution in CPPs modified with a ligand**

In the same way as in the case of CPPs containing no ligands, it is preferable that the CPPs comprising a ligand present a certain degree of helicity. Preferably, the CPP has a percentage of helicity in liposomes greater than 10%, as measured at 40°C in Milli-Q water in egg yolk phosphatidylcholine liposomes, and comprises a first half and a second half when separated by a plane containing the theoretical axis of the helix formed by the scaffold, wherein the first half comprises substantially all hydrophobic amino acids (e.g. alanine, leucine, isoleucine, valine, proline, methionine, phenylalanine or tryptophan), and the second half comprises substantially all the cationic amino acids (e.g. lysine, arginine or histidine), and the protein-binding units are located at the interface of said hydrophilic/hydrophobic domains. In this configuration the impact on the secondary structure is minimized.

Preferably, the scaffold of the CPP-ligand comprises at least two of the hydrophobic blocks each comprising one hydrophobic amino acid selected from leucine, isoleucine, valine, proline, methionine, phenylalanine and tryptophan. Preferably, the scaffold of the CPP-ligand comprises at least one cationic block comprising two cationic amino acids selected from lysine, arginine and histidine, preferably two arginine aminoacids.

The CPPs of the invention can comprise ligands which recognize and associate to specific molecules of biological interest for their transport into the cytosol.

The CPP of the invention can comprise a linker between the ligand and the peptidic scaffold. Said linker can include a hydrazone group or an oxime group. For example, the CPP of the invention can be one comprising one or more, for example one, two three or four, ligands (e.g. a monosaccharide or a polysaccharide) attached to the peptide through a linker comprising an oxime or a hydrozone. The oxime (or hydrazone) linker can be attached to any amino acids of the scaffold, typically, to the nitrogen of a lysine, or an arginine or a serine or a cysteine or an asparagine, more typically to a lysine. A typical linker can be of the formula $*-(C=O)-(CH_2)_n-O-N=C(H)-(CH_2)_m-\#$, wherein n and m are integers, preferably each independently 1, 2, 3 or 4, and wherein the end $*$ is attached to the peptidic scaffold, and the end $\#$ is attached to the ligand.

The ligands and molecules of biological interest can be chosen from a wide variety. The only requirement is that a ligand is available which binds to the molecule of biological interest. Exemplary molecules of biological interest are proteins, antibodies, polymers or mixtures thereof. The skilled person must only check the ligands which will bind to a given molecule of biological interest. These are even listed by providers. For example, in the case of lectins, the skilled person can readily check a list of ligands that can be found at the website of Interchim (http://www.interchim.fr/ft/M/MS902z.pdf), wherein the proteins of this family are classified according to their binding ligand. The present invention contains in examples 3.5-3.8 and 3.15 the pairs mannose/Concanavalin A and biotine/Streptavidin, but many more are available to the skilled person which should be effective with the CPPs of the present invention, such as monosaccharides (glucose, mannose), disaccharides (lactobionic acid...), oligosaccharides, polysaccharides, lipids, steroids, catecholamines, vitamins (biotin, folic acid...), nucleotides and their analogues, nucleosides and their analogues, nucleic acids and their analogues, enzyme cofactors, enzyme substrates, enzyme inhibitors, aminoacids and analogues, peptides and cyclic peptides, adamantanes, synthetic ligand analogues, or drugs.

Thus, in order to prove this technology, the inventors have prepared CPPs according to the invention terminated with a fluorophore (TAMRA) or by acetylation, and including a ligand, for example mannose or biotin (See examples 3.5-3.8, 3.15 or 3.16). CPPs comprising mannose were assayed for transport of Concavalin A (ConA), and CPPs comprising biotin were assayed for Streptavidin. Thus, the CPP of the invention preferably comprises as a ligand at least one monosaccharide or oligosaccharide, preferably at least one molecule of glucose or at least one molecule of mannose, more preferably, a monosaccharide or oligosaccharide comprising at least one molecule of glucose. For example, the CPP of the invention can be one comprising one or more, for example one, two three or four, glucose molecules attached to the peptide through a linker comprising an oxime or a hydrazone. The oxime linker can be attached to any amino acids of the scaffold, typically, to the nitrogen of a lysine, or an arginine or a serine or a cysteine or an asparagine, more typically to a lysine. A typical linker can be of the formula $*-(C=O)-(CH_2)_n-O-N=C(H)-(CH_2)_m-\#$, or formula $*-(CH_2)_n-CO-N-N=C(H)-(CH_2)_m-\#$, wherein n and m are integers, preferably each independently 1, 2, 3 or 4, and wherein the end $*$ is attached to the peptidic scaffold, and the end $\#$ is attached to the glucose molecule.

Alternatively, a vitamin such as biotin can be used as ligand.

**Preparation of CPPs**

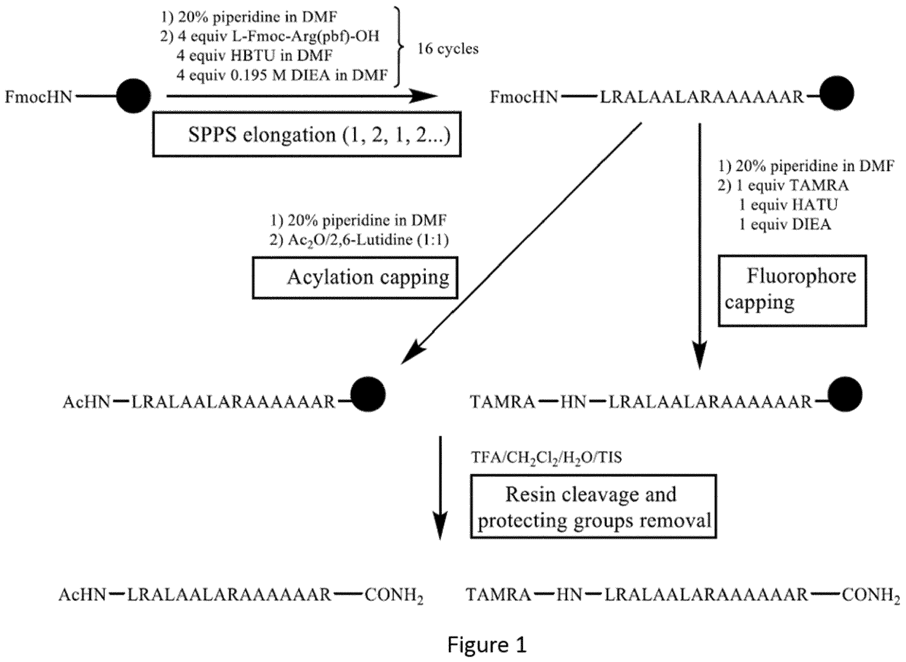

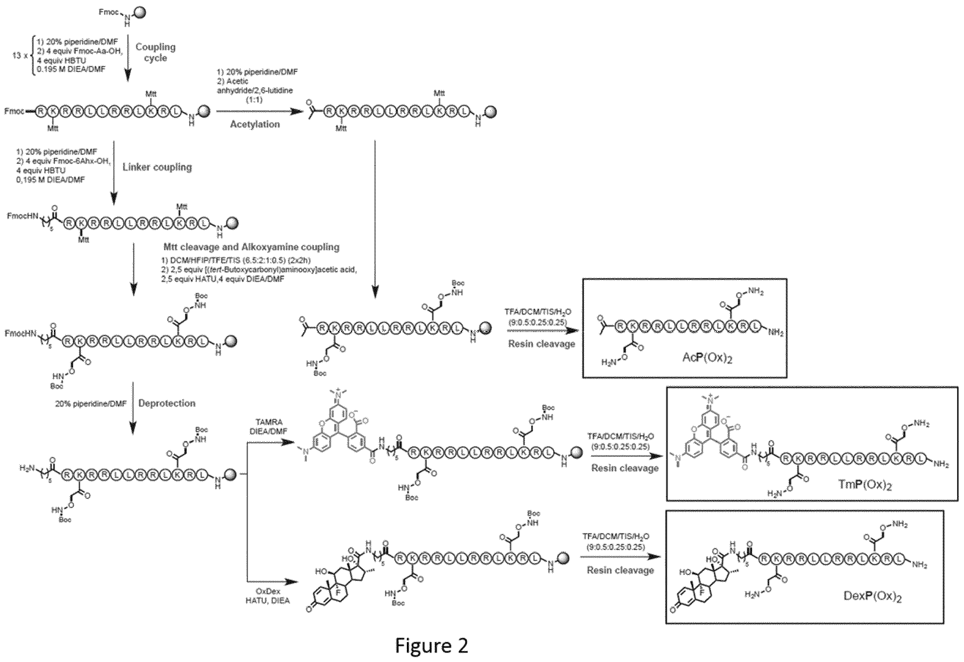

The synthesis of the CPPs was accomplished by known solid phase strategies using orthogonal protecting groups (Figures 1 and 2, and examples 1.1 and 3.1). The scaffold was thus prepared by successive cycles of deprotection-coupling. The peptide was terminated with either a fluorophore (TAMRA), the steroid dexamethasone or by simple acetylation. Where needed for ligand coupling, selective cleavage of methyltrityl (Mtt) groups of lysines proceeded in weakly acidic conditions and the reactive alkoxyamines were then coupled 'on resin' for the subsequent incorporation of aldehydes or ketones. Further modifications also followed known protocols. Full deprotection and cleavage from the solid support was accomplished by TFA treatment and, after precipitation and washings, the peptide was re-dissolved in water and fused to the protein ligands by a short incubation with the mannosyl or the glucosyl aldehyde (5 minutes, quantitative). After HPLC purification the peptide was characterized by circular dichroism (CD).

## EXAMPLES

## Materials and methods

A **microwave assisted peptide synthesiser** (Liberty Lite, CEM) was used to prepare the peptides according to standard methods developed by the manufacturers involving diisopropylcarbodiimide (DIC) 0.5 M in DMF as activator and ethyl(hydroxyimino)cyanoacetate (Oxyma) 1 M in DMF as activator base. Previous to HPLC, a quick size exclusion chromatography in sephadex G-25 eluted with Milli-Q water was carried out to remove the excess of fluorophore.

**High-performance liquid chromatography coupled with mass spectrometry (HPLC-MS) analyses** were carried out on Agilent Technologies 1260 Infinity II associated with a 6120 Quadrupole LC-MS using an Agilent SB-C18 column or on DIONEX Ultimate 3000 U-HPLC$^+$ (Thermo Scientific) with an Acclaim RSLC 120-C18 column with *Solvent A:Solvent B* gradients between 5:95 (*Solvent A:* $H_2O$ with 0.1% TFA; *Solvent B:* $CH_3CN$ with 0.1% TFA).

**High-performance liquid chromatography (HPLC) semi-preparative** purification was carried out on Jasco LC-4000 with an Agilent Eclipse XDB-C18 column. High-performance liquid chromatography (HPLC) preparative purification was carried out on Waters 1525 composed by a binary pump with a dual Waters 2489 detector with a Phenomenex Luna C18(2) 100A column. An Agilent 1200 with an Agilent Eclipse XDB-C18 column was used for semi-preparative purification using gradients between 5:95 and 5:75 (*Solvent A:* $H_2O$ with 0.1 % TFA; *Solvent B:* $CH_3CN$ with 0.1% TFA).

**Circular Dichroism (CD)** measurements were performed with a Jasco J-1100 CD Spectrometer equipped with a Jasco MCB-100 Mini Circulation Bath for temperature control.

A Tecan Infinite F200Pro microplate reader was used to measure directly in Costar cell culture 96-well plates UV-Vis absorbance for the **MTT viability assays.**

**Flow cytometry** was performed on a Guava easyCyte™ cytometer. Data analysis was performed with InCyte software included in GuavaSoft 3.2 (Millipore).

## Abbreviations

Peptide Abbreviations: TmP(Man)$_2$ (Tm = TAMRA and Man = Mannose aldehyde); Arg: Arginine; BSA: Bovine Serum Albumin; Boc: *tert*-Butoxycarbonyl; Calcd: Calculated; CF: 5(6)-Carboxyfluorescein; ConA: Concanavalin A; DCM: Dichloromethane; DIC: diisopropylcarbodiimide; DIEA: *N,N*-Diisopropylethylamine; DMAP: 4-Dimethylaminopyridine; DMEM: Dulbecco's Modified Eagle Medium; DMF: *N,N*-Dimethylformamide; DMSO: Dimethylsulfoxide; EYPC: Egg yolk phosphatidylcholine; FBS: Fetal Bovine Serum; Fmoc: 9-fluorenylmethoxycarbonyl; GFP: Green Fluorescent Protein; HFIP: 1,1,1,3,3,3-Hexafluoro-2-propanol; HKR: HEPES-Krebs-Ringer buffer; Leu: Leucine; Lys: Lysine; Mtt: 4-Methyltrytil; MTT: 3-(4,5-dimethylthiazol-2-yl)-2,5-diphenyltetrazolium bromide; *N*-HATU:*N*-[(Dimethylamino)-1H-1,2,3-triazolo[4,5-b]pyridine-lylmethylene]-*N*-methylmethanaminium-hexafluorophosphate N-oxide; *N*-HBTU: *N*-[(1HBenzotriazol-1-yl)-(dimethylamino)methylene]-*N*-methylmethanaminium hexafluorophosphate N-oxide; PI: propidium iodide; RP: Reverse Phase; SPPS: solid phase peptide synthesis; TAMRA: 5-carboxytetramethyl rhodamine; TFE: Trifluoroethanol; TNBS: 2,4,6-Trinitrobenzenesulfonic acid; TIS: Triisopropylsilane;

## General protocol to measure Circular Dichroism

Circular dichroism measurements were carried out with the following settings: acquisition range: 300-190 nm; band width: 1.0 nm; accumulation: 3 scans; data pitch: 1 nm; CD scale 200 mdeg/1.0 dOD; D.I.T. (Data Integration Time): 1 s; scanning mode: continuous; scanning speed: 200 nm/min. Measurements were done from 10 ºC to 60 ºC (data interval: 10 ºC; temp. gradient 5 ºC/min) in a quartz cell of 0.2 cm path length at a final volume of 0.5 mL ($H_2O$ or TFE) with a final peptide concentration of 200 $\mu$M.

The results are expressed as the mean residue molar ellipticity $[\theta]_{MRt}$ with units of degrees·cm$^2$.dmol$^{-1}$ and calculated using the equation S1.

The percentage of helicity was calculated using the equation S2. For the measurements in liposomes, samples were prepared by drying under reduced pressure L-$\alpha$-phosphatidylcholine (16.7 $\mu$L, 25 mg/mL solution in CHCl$_3$) and peptide (300 $\mu$L, 100 $\mu$M) in TFE to obtain a ratio lipid/peptide of 18:1 in a 10 mL round bottomed flask. The mixture was concentrated in a rotary evaporator to dryness, forming a film. The lipid/peptide mixture were suspended in HKR buffer (300 μL) to a final peptide concentration 100μM and sonicated for 45 min until a clear solution was obtained. Spectra were recorded in a 0.2 cm path length quartz cell.

$$[\Theta]_{MRt} = \frac{0.1\, x\, \Theta}{C \cdot l \cdot \text{number of residues}} \quad (\text{deg} \cdot \text{cm}^2 \cdot \text{dmol}^{-1}) \qquad (S1)$$

**Equation S1:** Formula to calculate the ellipticity. $\theta$ is the ellipticity (mdeg), C is the peptide concentration (M) and I is the cell path length (cm).

$$\text{helicity \%} = \frac{\Theta MRt\, 222nm - 2340}{30300} \; x\, 100 \qquad (S2)$$

**Equation S2:** Formula to calculate the percent of helicity in where the molar ellipticity at 222 nm is an absolute value.

### General protocols

### Example 1: General protocol 1 for the preparation of the CPPs

Example 1.1: SPPS Elongation

The general procedure for protocol 1 is summarized in Figure 1.

According to protocol 1, peptides were synthesized by automated or manual Fmoc solid-phase peptide synthesis using Rink Amide ChemMatrix resin (loading 0.5 mmol/g). For manual synthesis, the resin (0.5 mmol) was swelled in DMF (peptide synthesis grade, 2 mL) for 20 min in a peptide synthesis vessel prior to synthesis. Coupling cycle comprised the removal of Fmoc protecting group with a solution of piperidine in DMF (20%, 2 mL) for 10 min and then the mixture was filtered and the resin was washed with DMF (3 x 2 mL, 1 min). The amino acid coupling was carried out by treatment with a solution of $\alpha$-amino acids (4 equiv.), *N*-HBTU (3.95 equiv.) in DMF (2 mL), which was mixed with DIEA (0.195 M solution in DMF, 1.2 equiv.) 1 min before the addition and the resulting mixture was shaken by bubbling Ar for 15 min. Finally, the resin was washed with DMF (3 x 2 mL, 1 min). The efficiency of each amino acid coupling and deprotection was monitored employing the TNBS test.

For automated synthesis, a variant of the previous protocol was used instead, according to manufacturer's recommendations. 0.05 mmol of Rink Amide resin was placed into the peptide synthesiser reaction vessel, swollen in DMF, followed by cycles of Fmoc cleavage with piperidine 20% in DMF, washings (3 x 5 mL), then amino acid (5 equiv. 2M amino acid solution in DMF), DIC (10 equiv.) and Oxyma (10 equiv.) were added into the reaction vessel and microwaved for 5 min under temperature control followed by washings (3 x 5 mL). All steps were performed under nitrogen atmosphere. After the linear peptide was finished the resin was transferred to a different reaction vessel to perform the peptide modification manually.

**6-aminohexanoic coupling:** after Fmoc cleavage with piperidine/DMF (20%, 2 mL), the linear peptide was treated with a solution of N-Fmoc-6-aminohexanoic acid (4 equiv.), *N*-HBTU (3.95 equiv.) and DIEA (0.195 M solution in DMF, 1.2 equiv.) in DMF.

**Acetylation:** after Fmoc cleavage with piperidine/DMF (20 %, 2 mL), the linear peptide was treated with a solution of 2,6-lutidine (1mL) and acetic anhydride (1mL) in DMF.

Example 1.2: Fluorophore Coupling

The Fmoc-protecting group of the lineal final peptide (or the previously attached linker) was removed by using a solution of piperidine in DMF (20%, 4 mL) for 15 min and the resin was washed with DMF (3 x 3 mL). The coupling was carried out by the addition of a solution of 5-Carboxytetramethylrhodamine (1 equiv.) and DIEA (0.195 M, 1 equiv.) in DMF (2 mL) and the mixture was stirred by bubbling Ar for 4 hours. Finally, the resin was washed with DMF (3 x 3 mL) and DCM (3 x 3 mL).

Example 1.3: General procedure of resin cleavage and protecting groups removal and purification

Finally, peptides were deprotected and cleaved from the resin by standard TFA cleavage procedure at rt by using the TFA/DCM/H$_2$O/TIS (90:5:2.5:2.5, 3 mL per 70 mg of resin) for 2 h. Then, the mixture was filtered, washed with TFA (1 mL) and the peptide was precipitated with ice-cold Et$_2$O (50 mL). The precipitate was centrifuged and dissolved in H$_2$O (5 mL).

Peptides were purified first with sephadex size exclusion chromatography (G-25, Milli-Q water) and then by semi-preparative high-performance liquid chromatography (HPLC) as previously described. Finally, the corresponding fractions were lyophilised to afford the pure peptide as colourless or pink solids.

Using the methods of the general protocol 1, the following peptides where prepared:

Example 1.4: Synthesis of peptide P$_1$ (comparative) SEQ ID NO: 1

Following the general protocol of the SPPS followed by fluorophore coupling, P$_1$ was obtained after RP-HPLC purification [Phenomenex Luna C18(2) 100A column, H$_2$O (0.1% TFA)/ CH$_3$CN (0.1% TFA) 95:5→5:95 (0→5 min), 95:5→5:95 (5→35 min)] with an overall yield of 12%. $R_t$ 13.0 min [RP-HPLC Agilent SB-C18 column, H$_2$O (0.1% TFA)/ CH$_3$CN (0.1% TFA) 95:5→5:95 (0→20 min)].

Example 1.5: Synthesis of peptide P$_2$ (SEQ ID NO: 2)

Following the general protocol of the SPPS followed by fluorophore coupling, **P$_2$** was obtained after RP-HPLC purification [Phenomenex Luna C18(2) 100A column, H$_2$O (0.1% TFA)/ CH$_3$CN (0.1% TFA) 95:5→5:95 (0→5 min), 95:5→5:95 (5→35 min)] with an overall yield of 10.7%. $R_t$ 14.6 min [RP-HPLC Agilent SB-C18 column, H$_2$O (0.1% TFA)/ CH$_3$CN (0.1% TFA) 95:5→5:95 (0→20 min)].

Example 1.6: Synthesis of peptide P$_3$ (SEQ ID NO: 3)

Following the general protocol of the SPPS followed by linker and fluorophore coupling, **P$_3$** was obtained after RP-HPLC purification [Phenomenex Luna C18(2) 100A column, H$_2$O (0.1% TFA)/ CH$_3$CN (0.1% TFA) 95:5→5:95 (0→5 min), 95:5→5:95 (5→35 min)] with an overall yield of 8.7%. $R_t$ 14.2 min. [RP-HPLC Agilent SB-C18 column, H$_2$O (0.1% TFA)/ CH$_3$CN (0.1% TFA) 95:5→5:95 (0→20 min)].

Example 1.7: Synthesis of peptide P$_4$ (SEQ ID NO: 4)

Following the general protocol of the SPPS followed by fluorophore coupling, **P$_4$** was obtained after RP-HPLC purification [Phenomenex Luna C18(2) 100A column, H$_2$O (0.1% TFA)/ CH$_3$CN (0.1% TFA) 95:5→5:95 (0→5 min), 95:5→5:95 (5→35 min)] with an overall yield of 6.5%. $R_t$ 14.9 min [RP-HPLC Agilent SB-C18 column, H$_2$O (0.1% TFA)/ CH$_3$CN (0.1% TFA) 95:5→5:95 (0→20 min)].

Example 1.8: Synthesis of peptide P$_5$ (SEQ ID NO: 5)

Following the general protocol of the SPPS followed by linker and fluorophore coupling, **P$_5$** was obtained after RP-HPLC purification [Phenomenex Luna C18(2) 100A column, H$_2$O (0.1% TFA)/ CH$_3$CN (0.1% TFA) 95:5→5:95 (0→5 min), 95:5→5:95 (5→35 min)] with an overall yield of 2.9%. $R_t$ 15.2 min [RP-HPLC Agilent SB-C18 column, H$_2$O (0.1% TFA)/ CH$_3$CN (0.1% TFA) 95:5→5:95 (0→20 min)].

Example 1.9: Synthesis of peptide P$_6$ (SEQ ID NO: 6)

Following the general protocol of the SPPS followed by linker and fluorophore coupling, **P$_6$** was obtained after RP-HPLC purification [Phenomenex Luna C18(2) 100A column, H$_2$O (0.1% TFA)/ CH$_3$CN (0.1% TFA) 95:5→5:95 (0→5 min), 95:5→5:95 (5→35 min)] with an overall yield of 6.4%. $R_t$ 15.2 min [RP-HPLC Agilent SB-C18 column, H$_2$O (0.1% TFA)/ CH$_3$CN (0.1% TFA) 95:5→5:95 (0→20 min)].

Example 1.10: Synthesis of peptide P$_7$ (SEQ ID NO: 7)

Following the general protocol of the SPPS followed by fluorophore coupling, **P$_7$** was obtained after RP-HPLC purification [Phenomenex Luna C18(2) 100A column, H$_2$O (0.1% TFA)/ CH$_3$CN (0.1% TFA) 95:5→5:95 (0→5 min), 95:5→5:95 (5→35 min)] with an overall yield of 5.3%. $R_t$ 15.5 min [RP-HPLC Agilent SB-C18 column, H$_2$O (0.1% TFA)/ CH$_3$CN (0.1% TFA) 95:5→5:95 (0→20 min)].

Example 1.11: Synthesis of peptide P$_8$ (SEQ ID NO: 8)

Following the general protocol of the SPPS followed by fluorophore coupling. **P$_8$** was obtained after RP-HPLC purification [Phenomenex Luna C18(2) 100A column, H$_2$O (0.1% TFA)/ CH$_3$CN (0.1% TFA) 95:5→5:95 (0→5 min), 95:5→5:95 (5→35 min)] with an overall yield of 1.7%. $R_t$ 14.6 min [RP-HPLC Agilent SB-C18 column, H$_2$O (0.1% TFA)/ CH$_3$CN (0.1% TFA) 95:5→5:95 (0→20 min)].

Example 1.12: Synthesis of peptide acetylated P$_8$ (SEQ ID NO: 9)

Following the general protocol 1, the acetylated **P$_8$** (AcL R ALAALA R A$_6$ R) was obtained after RP-HPLC purification [Phenomenex Luna C18(2) 100A column, H$_2$O (0.1% TFA)/ CH$_3$CN (0.1% TFA) 95:5→5:95 (0→5 min), 95:5→5:95 (5→35 min)] with an overall yield of 1.7%. $R_t$ 14.6 min [RP-HPLC Agilent SB-C18 column, H$_2$O (0.1% TFA)/ CH$_3$CN (0.1% TFA) 95:5→5:95 (0→20 min)].

Example 1.13: Synthesis of peptide R$_8$ (comparative)

Following the general protocol of the SPPS followed by fluorophore coupling. **R$_8$** was obtained after RP-HPLC purification [Phenomenex Luna C18(2) 100A column, H$_2$O (0.1% TFA)/ CH$_3$CN (0.1% TFA) 100:0→100:0 (0→5 min), 50:50→50:50 (5→35 min)] with an overall yield of 1.7%. $R_t$ 14.1 min [RP-HPLC Agilent SB-C18 column, H$_2$O (0.1% TFA)/ CH$_3$CN (0.1% TFA) 95:5→5:95 (0→20min).

Example 1.14: Synthesis of acetylated peptide R8 (comparative)

Following the general protocol of the SPPS, acetylated R8 was obtained after RP-HPLC purification [Phenomenex Luna C18(2) 100A column, H$_2$O (0.1% TFA)/ CH$_3$CN (0.1% TFA) 100:0→100:0 (0→5 min), 50:50→50:50 (5→35 min)] with an overall yield of 1.7%. Rt 8.2 min [RP-HPLC Agilent SB-C18 column, H$_2$O (0.1% TFA)/ CH$_3$CN (0.1% TFA) 95:5→5:95 (0→20min).

**Example 2: Cell assays for the CPPs obtained according to protocol 1**

Example 2.1: Cell culture

HeLa and A549 cells were grown at 37 ºC, 5% CO$_2$, in Dulbecco's Modified Eagle's Medium (4500 mg/L glucose, L-glutamine, sodium pyruvate and sodium bicarbonate; SigmaAldrich), supplemented with 10% fetal bovine serum (Sigma-Aldrich) and 1% of Penicillin-Streptomycin-Glutamine Mix.
Sf9 Cells were grown at 28 ºC, in mechanical stirring, in Gibco Sf-900-II medium, complemented with 10% fetal bovine serum (Sigma-Aldrich) and 1% of Penicillin-Streptomycin-Glutamine Mix.

Example 2.2: Quantification of the uptake by flow cytometry

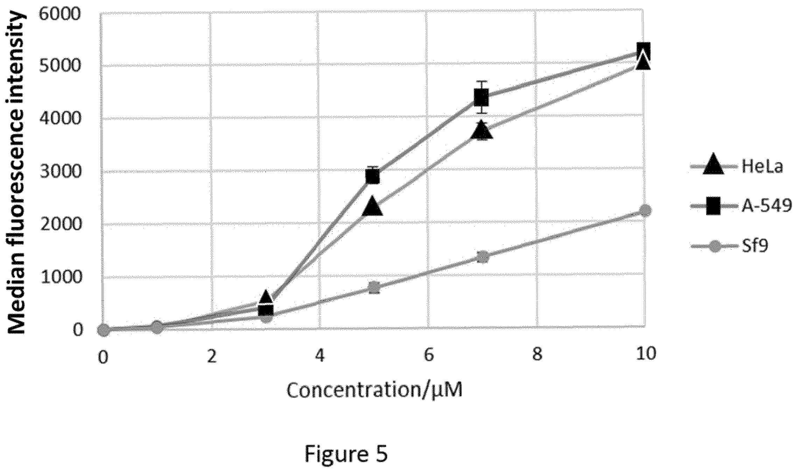

For internalization standard assays we measured the amount of peptide fluorescence in cells by flow cytometry. Briefly, semiconfluent monolayers of HeLa cells seeded the day before were washed twice with HKR before incubation with different concentrations (1, 3, 5, 7, 10 µM) of TAMRA labelled peptides diluted in cell culture medium without serum for 1 hour at 37 ºC . Then, cells were trypsinized with 100 µL of Trypsin-EDTA (Gibco) for 10 min at 37 ºC, and trypsin neutralized with 100 µL of 2 % FBS in PBS with 5 mM EDTA. Pipetting broke cell clumps and the analysis was done in a Guava easyCyte™ cytometer. TAMRA levels were determined by excitation at 532 nm and detection at 575/25 nm. For the analysis, cells with typical FSC and SSC parameters were selected, and median fluorescence intensity was calculated for each sample. Data analysis was performed with InCyte software included in GuavaSoft 3.2 (Millipore).
The results of uptake in living HeLa cells for P$_1$ (comparative), and P$_2$ to P$_8$ are shown in Figure 3. The results of uptake of CPP P$_8$ in HeLa, A-549 and SF9 are depicted in Figure 5: Uptake quantification by flow cytometry in three different cell lines: HeLa cells, A549 cells and Sf9 cells. Data points were collected in triplicate at six different concentrations (0, 1, 3, 5, 7, 10 µM). Cells were treated in DMEM (-) (HeLa, A549), or Sf-900-II (-) (Sf9) for 1 hour prior to trypsinization and cytometry quantification. Error bars indicate SD of three replicates.

Example 2.3: Dextran escape from endosomes

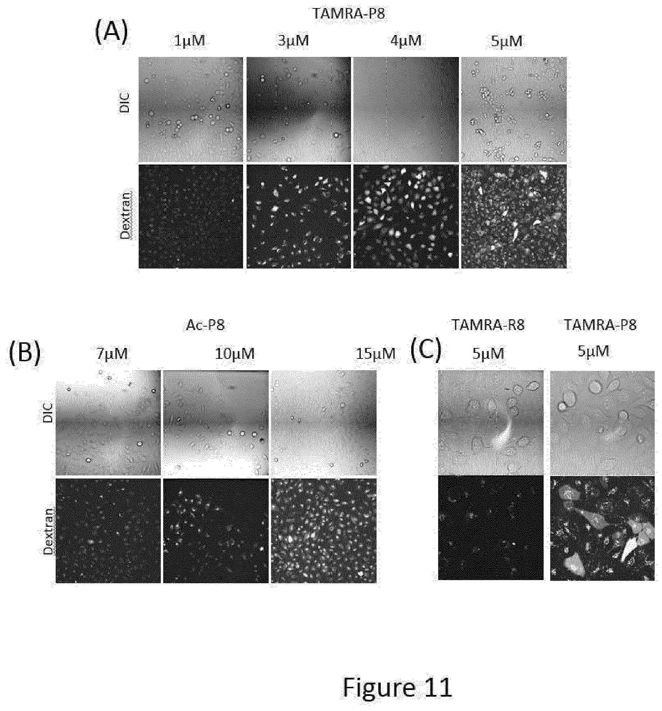

Dextran is a polysaccharide (in this case labeled with a green fluorescent probe) known to internalize cells through the endocytic pathway. Thus, if the CPPs of the invention are capable of inducing release of dextrane from endosomes into the cytosol, the cell will display diffuse green fluorescence. The experiment was run by incubating dextran (250 $\mu$g/ml) in the presence of 5 $\mu$M of a CPP: (i) $P_8$ marked with TAMRA, (ii) acetylated $P_8$, and (iii) $R_8$ as comparison. The results are shown in Figure 11.

The results show that the peptide TAMRA-P8 (Figure 11A) starts to release the dextran into the cytosol from 3 $\mu$M onwards, while the acetylated P8 starts at 10 $\mu$M (Figure 11B). However, incubation with TAMRA-R8 at 5 $\mu$M does not have any effect on the release of the dextran, that remains trapped in the endosomes (Figure 11C).

Example 2.4: Viability in the presence of Saporin

Saporin (253 aa, 28 kDa) is a toxic protein from the group of the ribosome-inactivating proteins (RIP), which is not capable of reaching the cytosol by itself, although it can be passively internalized by pinocytosis if it is found in the culture medium, being retained in endosomes. Incubation of the cells with this protein in the presence of an endosomolytic agent causes the release of the protein to the cytosol and cell death.

For the experiment, HeLa cells were incubated in a 96-well plate with 10 $\mu$g/mL of saporin, and increasing concentrations of the $P_8$ and $R_8$ acetylated peptides.

The cells were co-incubated with the protein and peptide for 1 h in culture medium without serum or antibiotics, at which time the compounds were removed, washed and complete medium was added for 6 h, to allow the maturation of the endosomes and the escape of the toxin. At that time, MTT (tetrazolium salt) was added, cells were further incubated for 3 h, and the medium removed before solubilizing the formazan salt with DMSO. This viability test measures the reduction of MTT to formazan by mitochondrial activity, which can then be quantified by absorbance.

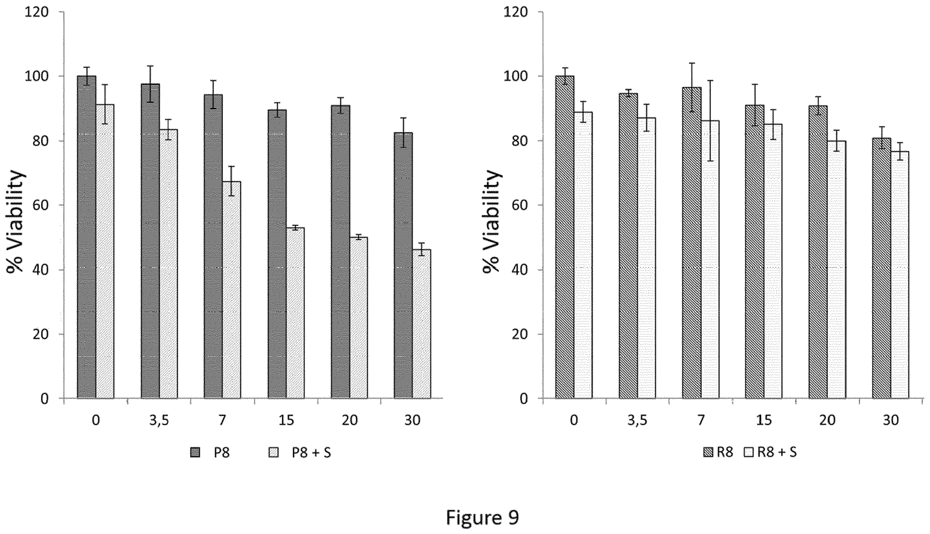

The results are summarized in Figure 9. The peptide $P_8$ alone presents little or no toxicity to HeLa cells up to 30 $\mu$M. However, in the presence of saporin, a reduction of viability is observed from 7 $\mu$M and higher concentrations, indicating that the peptide is releasing the toxin into the cytosol due to endosomal disruption. In the case of $R_8$, no increase of the toxicity with co-incubation with saporin was observed in that concentration range, indicating that $R_8$ is unable to release the toxin into the cytosol.

Example 2.5: Cre Recombinase protein Cytosolic delivery

In order to further test the capabilities of the CPPs of the invention to release macromolecules into the cytosol, a Cre recombinase delivery assay was done in the presence of $P_8$. HeLa cells stably transfected with a plasmid carrying the LoxP-DsRED-STOP-LoxP-eGFP insert (Cre Reporter system) were incubated with 1 $\mu$M Cre recombinase protein and CPPs $P_8$ or $R_8$ at different concentrations.

These cells express normally the dsRED protein (red fluorescence), but when the Cre recombinase protein removes the dsRED ORF by recombination, the eGFP gene (green fluorescence) is expressed instead. Thus, the percentage of cells expressing eGFP can be used as a measure of the percentage of cells where the Cre Recombinase had been effectively released into the cytosol.

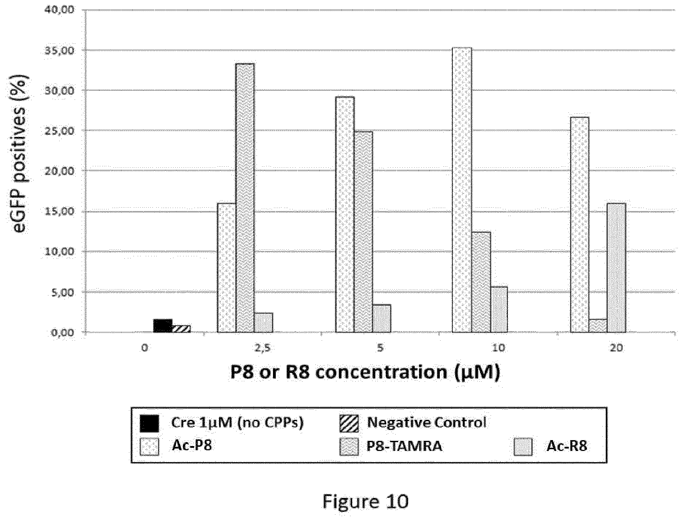

The results are shown in Figure 10. From the results it can be seen that TAMRA-P8 (Tm-P8) provides a maximum percentage of eGFP expressing cells of 33% at 2.5 $\mu$M and Acetylated-P8 (Ac-P8) a maximum of 35% at 10 $\mu$M, while acetylated-R8 (Ac-R8) only achieved a maximum of about 15% at 20 $\mu$M.

**Example 3: General protocol 2 for the preparation of the CPPs**

Example 3.1: SPPS Elongation or coupling cycle

The general procedure for protocol 2 is summarized in Figure 2.

According to protocol 2, peptides were synthesized by manual Fmoc solid-phase peptide synthesis using Rink Amide resin (loading 0.71 mmol/g). The resin (0.1 mmol) was swelled in DMF (peptide synthesis grade, 2 mL) for 20 min in a peptide synthesis vessel prior synthesis. Coupling cycle consisted of the removal of Fmoc protecting group with a solution of piperidine in DMF (20%, 2 mL) for 10 min and then the mixture was filtered and the resin was washed with DMF (3 x 2 mL, 1 min). The amino acid coupling was carried out by treatment with a solution of $\alpha$-amino acids (4 equiv), N-HBTU (3.95 equiv) in DMF (2 mL), which was mixed with DIEA (0.195 M solution in DMF, 1.2 equiv) 1 min before the addition and the resulting mixture was shaken by bubbling Ar for 15 min. Finally, the resin was washed with DMF (3 x 2 mL, 1 min). The efficiency of each amino acid coupling and deprotection was monitored employing the TNBS test.

Once the linear peptide was finished, two different ending protocols were used:

**Acetylation:** the acetylation capping of N-terminal group was performed by standard Fmoc removal conditions (20% piperidine in DMF) followed by treatment with a solution of acetic anhydride and 2,6-lutidine (1:1, 1 mL) for 30 min.

6-aminohexanoic acid coupling: after Fmoc cleavage with piperidine/DMF (20%, 2 mL), the linear peptide was treated with a solution of N-Fmoc-6-aminohexanoic acid (4 equiv), N-HBTU (3.95 equiv) and DIEA (0.195 M solution in DMF, 1.2 equiv) in DMF.

In either case the resin was washed with DCM (2 x 2 mL, 5 min), and the Mtt protecting group was selectively removed by mechanical shaking of the resin with a mixture of DCM/HFIP/TFE/TIS (6.5:2:1:0.5, 2 x 2 mL, 2 h). Finally, the mixture was filtered and the resin was washed with DCM (2 x 2 mL, 2 min) and DMF (2 mL, 20 min). A solution of [(*tert*-butoxycarbonyl)aminooxy]acetic acid or 5-(2-(tert-butoxycarbonyl)hydrazineyl)-5-oxopentanoic acid (2.5 equiv per free amine) and N-HATU (2.5 equiv) in DMF (1 mL) was added to the resin followed by the dropwise addition of a solution of DIEA (4 equiv) in DMF (0.5 mL). The resin was shaken by bubbling Ar for 30 min and finally washed with DMF (3 x 2 mL, 2 min) and DCM (3 x 2 mL, 2 min).

Example 3.2: General protocol for N-terminal functionalization

For fluorescently labelled peptides, the Fmoc-protecting group of the previously attached N-Fmoc-6-aminohexanoic acid was removed by using a solution of piperidine in DMF (20%, 4 mL) for 15 min and the resin was washed with DMF (3 x 3 mL). The coupling was carried out by the addition of a solution of 5(6)-Carboxytetramethylrhodamine succinimidyl ester (1 equiv) and DIEA (0.195 M, 1 equiv) in DMF (2 mL) and the mixture was stirred by bubbling Ar for 30 min. Finally, the resin was washed with DMF (3 x 3 mL) and DCM (3 x 3 mL).

For Ox-Dex terminating peptide, the Fmoc-protecting group of the linker was removed by using a solution of piperidine in DMF (20%, 4 mL) during 15 min and the resin was washed with DMF (3 x 3 mL). A solution of Ox-Dex (3 equiv), N-HATU (2.9 equiv) and DIEA (0.195 M, 3 equiv) in DMF (2 mL) was added and the mixture was shaken by bubbling Ar for 30 min. Finally, the resin was washed with DMF (3 x 3 mL) and DCM (3 x 3 mL).

Example 3.3: General protocol for peptide cleavage

Finally, peptides were deprotected and cleaved from the resin by standard TFA cleavage procedure at rt by using TFA/DCM/$H_2$O/TIS (90:5:2.5:2.5, 1 mL per 70 mg of resin) for 2 h. Then, the mixture was filtered, washed with TFA (1 mL) and the peptide was precipitated with ice-cold $Et_2$O (25 mL). The precipitate was centrifuged and dissolved in $H_2$O (5 mL).

Peptides obtained following protocol 2 were treated with the different ligands without purification.

Example 3.4: General protocol for ligand coupling (not shown in Figure 2)

A solution of peptide (XP(Ox)$_2$) (X being Tm or Ac) in $H_2$O (5 mM) was reacted with a solution of corresponding ligands (2 equiv per alkoxyamine) [1-α-formylmethyl-mannopyranoside or acetone] in $H_2$O (120 mM) for 5 min. Then, peptides were purified by RP-HPLC for removing the ligand excess.

The purification was carried by a C18 RP-HPLC [Phenomenex Luna C18(2) 100A column, $H_2$O (0.1% TFA)/$CH_3$CN (0.1% TFA) 95:5→5:95 (0→5 min), 95:5→5:95 (5→35 min)] with a binary gradient of *Solvent A* and *Solvent B (Solvent A:* $H_2$O with 0.1% TFA; *Solvent B:* $CH_3$CN with 0.1% TFA), the collected fractions were lyophilized and stored at -20 °C. Purity and identity were confirmed by analytical HPLC, $^1$H-NMR and low and high resolution mass spectrometry.

A solution of peptide (XP(Hyd)$_2$) (X being Tm or Ac) in DMSO (2.5 mM) was mixed with 6 equiv of the aldehyde tail (or aldehyde modified ligand) per hydrazide dissolved in DMSO in the presence of 5 % of AcOH and shaken at 60 ºC for 2 h, without further purification. Concentration of the peptide in the reaction was 1.25 mM. Before incubation with the complexes, reactions were diluted to 50 μM using DMEM or PBS.

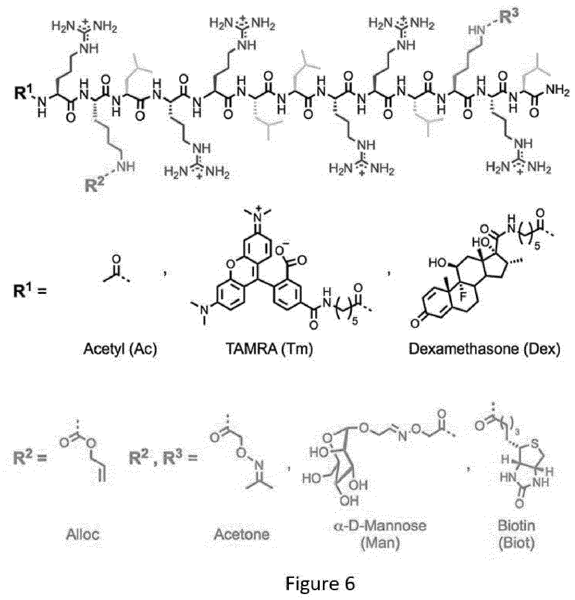

Using the methods of the general protocol 2, the following CPPs where prepared. The CPPs follow the formula $R_1$-P-($R_2$)($R_3$), wherein $R_1$ can be Ac (acetyl) or Tm (TAMRA), P is the peptide having the sequence RKLRRLLRRLKRL, and each of $R_2$ and $R_3$ is a group attached to a lysine unit of P, selected from Alloc (-C(=O)-O-(CH$_2$)-(CH)=(CH$_2$)), acetone (-(C=O)-(CH$_2$)-O-N=C(CH$_3$)$_2$), Man (-(C=O)-(CH$_2$)-ON=(CH)-(CH$_2$)-alpha-D-Mannose) and Biot (-(C=O)-(CH$_2$)$_4$-biotin). See Figure 6.

**Example 3.5: Synthesis of AcP(Man)$_2$** (SEQ ID NO: 10)

Following the general protocol of the SPPS for synthesizing an acetylated peptide with two mannoses, AcP(Man)$_2$ was obtained after RP-HPLC purification [Phenomenex Luna C18(2) 100A column, $H_2$O (0.1% TFA)/ $CH_3$CN (0.1% TFA) 95:5→5:95 (0→5 min), 95:5→5:95 (5→35 min)] with an overall yield of 28% and 99.3% purity. $R_t$ 3.8 min [RP-HPLC Agilent SB-C18 column, $H_2$O (0.1% TFA)/ CH3CN (0.1% TFA) 95:5→5:95 (0→5 min)].

**Example 3.6: Synthesis of TmP(Man)$_2$** (SEQ ID NO: 11)

Following the general protocol of the SPPS for synthesizing a TAMRA labelled peptide with two mannoses,

TmP(Man)$_2$ was obtained after RP-HPLC purification [Phenomenex Luna C18(2) 100A column, H$_2$O (0.1% TFA)/ CH$_3$CN (0.1% TFA) 95:5→5:95 (0→5 min), 95:5→5:95 (5→35 min)] with an overall yield of 6% and 100% purity. $R_t$ 4.1 min [RP-HPLC Agilent SB-C18 column, H$_2$O (0.1% TFA)/ CH$_3$CN (0.1% TFA) 95:5→5:95 (0→5 min)].

Example 3.7: Synthesis of AcP(Alloc)(Man) (<u>SEQ ID NO: 12</u>)

Following the general protocol of the SPPS for synthesizing an acetylated peptide with one mannose, and using Alloc protected Lysine, Ac**P**(Alloc)(Man) was obtained after RP-HPLC purification [Phenomenex Luna C18(2) 100A column, H$_2$O (0.1% TFA)/ CH$_3$CN (0.1% TFA) 95:5→5:95 (0→5 min), 95:5→5:95 (5→35 min)] with an overall yield of 15% and 100% purity. $R_t$ 4.1 min [RP-HPLC Agilent SB-C18 column, H$_2$O (0.1% TFA)/ CH$_3$CN (0.1% TFA) 95:5→5:95 (0→5 min)].

**Example 3.8: Synthesis of TmP(Alloc)(Man)** (<u>SEQ ID NO: 13</u>)

Following the general protocol of the SPPS for synthesizing a TAMRA labelled peptide with one mannose, and using Alloc protected Lysine, TmP(Alloc)(Man) was obtained after RP-HPLC purification [Phenomenex Luna C18(2) 100A column, H$_2$O (0.1% TFA)/ CH$_3$CN (0.1% TFA) 95:5→5:95 (0→5 min), 95:5→5:95 (5→35 min)] with an overall yield of 10% and 98.4% purity. $R_t$ 4.3 min [RP-HPLC Agilent SB-C18 column, H$_2$O (0.1% TFA)/ CH$_3$CN (0.1% TFA) 95:5→5:95 (0→5 min)].

**Example 3.9: Synthesis of AcP(Acetone)$_2$ (comparative)**

Following the general protocol of the SPPS for synthesizing an acetylated peptide capped with acetone, AcP(Acetone)$_2$ was obtained after RP-HPLC purification [Phenomenex Luna C18(2) 100A column, H$_2$O (0.1% TFA)/ CH$_3$CN (0.1% TFA) 95:5→5:95 (0→5 min), 95:5→5:95 (5→35 min)] with an overall yield of 4% and 100% purity. $R_t$ 4.3 min [RP-HPLC Agilent SB-C18 column, H$_2$O (0.1% TFA)/ CH$_3$CN (0.1% TFA) 95:5→5:95 (0→5 min)].

**Example 3.10: Synthesis of TmP(Acetone)$_2$ (comparative)**

Following the general protocol of the SPPS for synthesizing a TAMRA labelled peptide capped with acetone, TmP(Acetone)$_2$ was obtained after RP-HPLC purification [Phenomenex Luna C18(2) 100A column, H$_2$O (0.1% TFA)/ CH$_3$CN (0.1% TFA) 95:5→5:95 (0→5 min), 95:5→5:95 (5→35 min)] with an overall yield of 12% and 99.9% purity. $R_t$ 4.4 min [RP-HPLC Agilent SB-C18 column, H$_2$O (0.1% TFA)/ CH$_3$CN (0.1% TFA) 95:5→5:95 (0→5 min)].

**Example 3.11: Synthesis of TmArg$_2$(Man)$_2$ (comparative)**

Following the general protocol of the SPPS for synthesizing a TAMRA labelled tetrapeptide with two mannoses, TmArg$_2$(Man)$_2$ was obtained after RP-HPLC purification [Phenomenex Luna C18(2) 100A column, H$_2$O (0.1% TFA)/ CH$_3$CN (0.1% TFA) 95:5→5:95 (0→5 min), 95:5→5:95 (5→35 min)] with an overall yield of 26% and 100% purity. $R_t$ 3.8 min [RP-HPLC Agilent SB-C18 column, H$_2$O (0.1% TFA)/ CH$_3$CN (0.1% TFA) 95:5→5:95 (0→5 min)].

**Example 3.12: Synthesis of TmArg$_6$Gly$_5$(Man)$_2$ (comparative)**

Following the general protocol of the SPPS for synthesizing a TAMRA labelled peptide with two mannoses, TmArg$_6$Gly$_5$(Man)$_2$ was obtained after RP-HPLC purification [Phenomenex Luna C18(2) 100A column, H$_2$O (0.1% TFA)/ CH$_3$CN (0.1% TFA) 95:5→5:95 (0→5 min), 95:5→5:95 (5→35 min)] with an overall yield of 9% and 100% purity. $R_t$ 3.4 min [RP-HPLC Agilent SB-C18 column, H$_2$O (0.1% TFA)/ CH$_3$CN (0.1% TFA) 95:5→5:95 (0→5 min)].

**Example 3.13: Synthesis of TmArg$_6$(Man)$_2$ (comparative)**

Following the general protocol of the SPPS for synthesizing a TAMRA labelled peptide with two mannoses, TmArg$_6$(Man)$_2$ was obtained after RP-HPLC purification [Phenomenex Luna C18(2) 100A column, H$_2$O (0.1% TFA)/ CH$_3$CN (0.1% TFA) 95:5→5:95 (0→5 min), 95:5→5:95 (5→35 min)] with an overall yield of 9% and 100% purity. $R_t$ 3.4 min [RP-HPLC Agilent SB-C18 column, H$_2$O (0.1% TFA)/ CH$_3$CN (0.1% TFA) 95:5→5:95 (0→5 min)].

**Example 3.14: Synthesis of CFArg$_8$ (comparative)**

For the preparation of the control peptide CFArg$_8$, the carboxyfluorescein was coupled in the amino group of a lysine Mtt localized at the end of the peptide sequence. Therefore, following the general protocol of the SPPS for growing the peptide, the Mtt protecting group was selectively removed by mechanically stirring the resin with a mixture of DCM/HFIP/TFE/TIS (6.5:2:1:0.5, 2 x 1 mL per 70 mg of resin) for 2 h. Finally, the mixture was filtered and the resin was washed with DCM (2 x 2 mL, 2 min) and DMF (2 mL, 20 min). Then, a solution of 5(6)-carboxyfluorescein (2 equiv) and N-HBTU (2 equiv) in DMF (1 mL) was added to the vessel followed by the dropwise addition of DIEA (4 equiv). The resulting mixture was shaken by bubbling Ar for 30 min and finally the filtered resin was washed with DMF (3 x 2 ml, 2 min) and DCM (3 x 2 ml, 2 min). CFArg8 was obtained after RP-HPLC purification [Phenomenex Luna C18(2) 100A column, $H_2O$ (0.1% TFA)/ $CH_3CN$ (0.1% TFA) 95:5→5:95 (0→5 min), 95:5→5:95 (5→35 min)] with an overall yield of 11% and 100% purity. $R_t$ 3.2 min [RP-HPLC Agilent SB-C18 column, $H_2O$ (0.1% TFA)/ $CH_3CN$ (0.1% TFA) 95:5→5:95 (0→5 min)].

**Example 3.15: Synthesis of AcP(Biot)$_2$** (SEQ ID NO: 14)

For the preparation of the biotinylated peptide, D-(+)-Biotin was coupled in the amino group of the lysines Mtt localized at the peptidic sequence. Therefore, following the general protocol of the SPPS for growing the peptide, the Mtt protecting group was selectively removed by mechanically stirring the resin with a mixture of DCM/HFIP/TFE/TIS (6.5:2:1:0.5, 2 x 1 mL per 70 mg of resin) for 2 h. Finally, the mixture was filtered and the resin was washed with DCM (2 x 2 mL, 2 min) and DMF (2 mL, 20 min). Then, a solution of D-(+)-Biotin (2.5 equiv) and N-HATU (2.5 equiv) in DMF (1 mL) was added to the vessel followed by the drop wise addition of DIEA (4 equiv). The resulting mixture was shaken by bubbling Ar for 30 min and finally the filtered resin was washed with DMF (3 x 2 ml, 2 min) and DCM (3 x 2 ml, 2 min).

AcP(Biot)$_2$ was obtained after RP-HPLC purification [Phenomenex Luna C18(2) 100A column, $H_2O$ (0.1% TFA)/ $CH_3CN$ (0.1% TFA) 95:5→5:95 (0→5 min), 95:5→5:95 (5→35 min)] with an overall yield of 11% and 100% purity. $R_t$ 4.1 min [RP-HPLC Agilent SB-C18 column, $H_2O$ (0.1% TFA)/ $CH_3CN$ (0.1% TFA) 95:5→5:95 (0→5 min)].

**Example 3.16: Synthesis of DexP(Man)$_2$** (SEQ ID NO: 15)

Following the general protocol of the SPPS for synthesizing an Dex labelled peptide with two mannoses, DexP(Man)$_2$ was obtained after RP-HPLC purification [Phenomenex Luna C18(2) 100A column, $H_2O$ (0.1% TFA)/ $CH_3CN$ (0.1% TFA) 95:5→5:95 (0→5 min), 95:5→5:95 (5→35 min)] with an overall yield of 5% and 99.6% purity. Rt 4.2 min.

**Example 3.17: Synthesis of TmP(Glu)$_2$** (SEQ ID NO: 16)

Following the general protocol of the SPPS for synthesizing a TAMRA labelled peptide with two glucoses, TmP(Glu)$_2$ was obtained after RP-HPLC purification [Agilent Eclipse XDB-C18 5 μm column, $H_2O$ (0.1% TFA)/ $CH_3CN$ (0.1% TFA) 95:5→5:95 (0→5 min), 95:5→5:95 (5→35 min)] with an overall yield of 11% and 100% purity. $R_t$ 4.2 min [RP-HPLC Agilent SB-C18 column, $H_2O$ (0.1% TFA)/ $CH_3CN$ (0.1% TFA) 95:5→5:95 (0→5 min)].

**Example 4: Cells Lines and Culture for testing the CPPs obtained according to protocol 2**

HeLa and HepG2 cells were incubated at 37 ºC/ 5% $CO_2$/ 95% humidity in an INCO 108 incubator (Memmert) with Dulbecco's Modified Eagle's Medium (4500 mg/L glucose, L-glutamine, sodium pyruvate and sodium bicarbonate; Sigma-Aldrich), supplemented with 10% fetal bovine serum (Sigma-Aldrich) and 1% of Penicillin-Streptomycin-Glutamine Mix (Fisher).

Example 4.1: Cell transport experiments

HeLa cells growing on four chamber glass bottom dishes were washed with HEPES-Krebs-Ringer (HKR) buffer (5 mM HEPES, 137 mM NaCl, 2.68 mM KCl, 2.05 mM $MgCl_2$, 1.8 mM $CaCl_2$, pH 7.4) and nuclei were stained by incubation with 1 μM Hoechst 33342 in HKR for 30 min. The CPP and protein were diluted in HKR and incubated for 7 min at room temperature to allow the formation of the complexes. Cells were washed with HKR and incubated with the complexes for another 30 min. Cells were washed again to remove excess peptide before performing epifluorescence microscopy using a Nikon Eclipse Ti-E inverted microscope or confocal microscopy with a Leica SP5 microscope. Images were analyzed with ImageJ and the co-localization parameters (Manders coefficients) were calculated using the Coloc2 plugin selecting the Costes method of threshold regression.

The transport of ConA was tested in the presence of TmP(Man)$_2$ and CFArg$_8$ (CF= carboxyfluorescein and Arg$_8$ for octaarginine), a prototypical penetrating peptide, was used as comparison. As expected, CFArg$_8$ was internalized inside the cells while ConA (labelled with Alexa Fluor-647) remained at the cell membrane. However, when the transport experiment was repeated with the TmP(Man)$_2$ (according to the invention), the ConA cellular uptake was observed. Quantification of the peptide and protein co-localization gave good values (aprox. 0.6) for the Manders coefficients, as shown in Table 2.

Table 2

| Peptide | Manders' tM$_1$ (Peptide) | Manders' tM$_2$ (Protein) |
|---|---|---|
| CFArg$_8$ | 0.03 | 0.04 |
| TmP(Man)$_2$ | 0.66 | 0.63 |

By contrast, TmP(Acetone)$_2$ did not complex or transport ConA. Further, to demonstrate that ConA binding and clustering was not sufficient for protein transport, control experiments were carried out with two different peptides not according to the invention with the same number of arginines, but containing the ligand mannose: (TmArg$_6$Gly$_5$(Man)$_2$ and TmArg$_6$(Man)$_2$). Also, a short cationic peptide (TmArg$_2$(Man)$_2$) was tested. All of them bearing two mannose residues. The results once again confirmed that the ConA could not go beyond the cell membrane.

In order to demonstrate protein discrimination, transport experiments with ConA$_{FITC}$ were carried out in the presence of a second labelled protein, namely Streptavidin$_{594}$ (Alexa Fluor-594). As expected, the CPP complexed with the ConA and the CPP/protein aggregates only contain the lectin. Analogous control transport experiments were performed with a commercially available nano-vehicle, Lipofectamine 2000, which is known to be a suitable excellent vehicle for negatively charged proteins. These experiments showed that the Lipofectamine transported the ConA and the Streptavidin without selectivity.

We next exchanged the glycan unit for a biotin ligand to explore the extension of the methodology to different proteins. Incubation of the biotinylated peptide (AcP(Biot)$_2$) with fluorescently labelled Streptavidin and subsequent cell transport experiments confirmed the presence of internalized Streptavidin as shown in the fluorescence confocal micrographs. Finally, protein transport experiments in HepG2 cells were performed to explore the extension of the method to different cell lines. These transport experiments, in a different cell line, showed similar results as the previously observed with HeLa cells. In this case, the protein targets (ConA and Streptavidin) were incubated with HepG2 cells in the presence (or absence) of the CPPs of the invention bearing the corresponding mannose and biotin ligands. On the other hand, in the absence of CPPs ConA remained in the membrane and a total lack of protein signal for the Streptavidin was observed.

Example 4.2: Cell viability

*Example 4.2.1: Cell viability: MTT Assay*

Cell viability was established by a standard MTT assay (Figure 7 and Figure 8). One day before the assay, a suspension of HeLa or HepG2 cells was plated in 96-well tissue culture plates (Costar 96 Flat Bottom Transparent Polystyrol) by adding 100 $\mu$L (150.000 cells/mL) per well. The next day, the medium was aspirated and cells were incubated with different concentrations of CPP and CPP/protein complexes diluted in HKR (50 $\mu$L/well). For acute toxicity studies, after 30 min of incubation at 37 °C, the medium was aspirated and replaced by fresh medium (DMEM) containing 10% FBS (100 $\mu$L). Control cells were given only cell culture medium (100 $\mu$L final medium). The viability was measured by quantifying the cellular ability to reduce the water-soluble tetrazolium dye 3-(4,5-dimethyl-2-thiazolyl)-2,5-diphenyl tetrazolium bromide (MTT) to its insoluble formazan salt as follows. MTT (5 mg/mL in PBS, 10 $\mu$L/well) was added to the wells and the cells were further incubated for 6 h. The supernatant was carefully removed and the water-insoluble formazan salt was dissolved in DMSO (100 $\mu$L/well). The absorbance was measured at 570 nm using a microplate reader (Infinite F200pro, Tecan). Data points were collected in triplicate and expressed as normalized values for untreated control cells (100%).

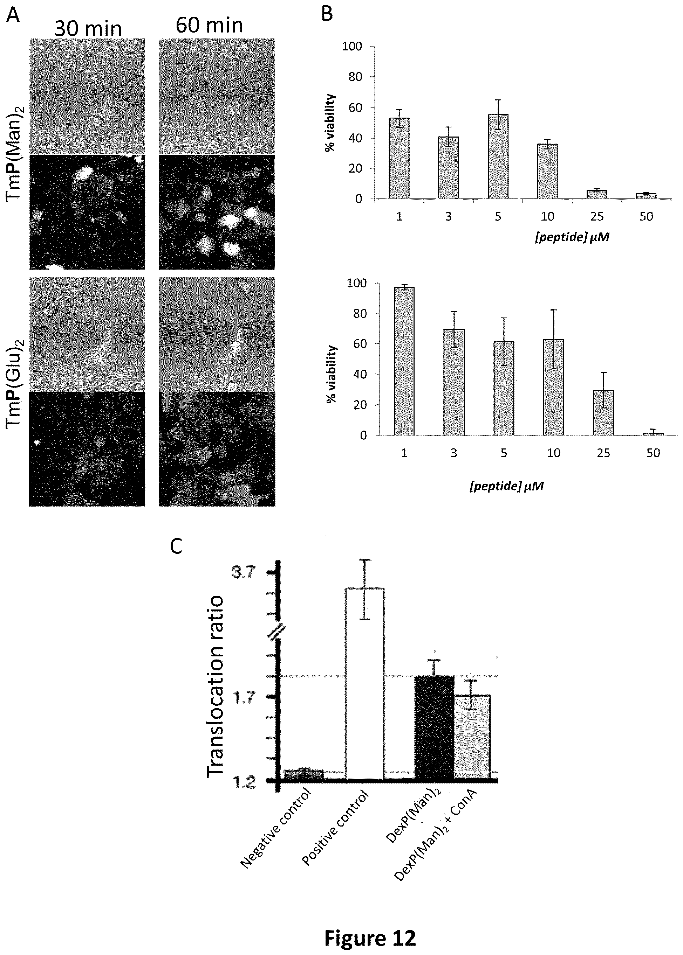

For medium term toxicity studies (Figure 12B), HeLa cells growing in 96 well plates were incubated with different concentration of the peptides diluted in HKR for 1 h, before washing twice with HKR and adding 100 $\mu$L of DMEM supplemented with 10 % FBS and antibiotics. Cells were incubated for three additional hours before the addition of MTT (10 uL of 5 mg/mL) and incubation for 3 h in the presence of MTT before lysis with DMSO and absorbance measurement.

*Example 4.2.2: Propidium Iodide Assay*

Cell viability was established by a standard PI assay (Figure 7 and Figure 8). One day before the assay, a suspension of HeLa or HepG2 cells was plated in 96-well tissue culture plates (Costar 96 Flat Bottom Transparent Polystyrol) by adding 100 $\mu$L (150.000 cells/mL) per well. The next day, the medium was aspirated and cells were incubated with different concentrations of peptide and peptide/protein complexes diluted in DMEM (50 $\mu$L/well). After 30 min of incubation at 37 °C, the medium was aspirated; cells were washed with fresh medium and then 100 $\mu$L of trypsin were added in each well and cells were incubated for 15 min at 37 °C. After this, 100 $\mu$L of a solution of 2% FBS and 5 mM EDTA in PBS containing PI (0.25 $\mu$g/mL) were added and the plate was incubated for 5 min in the dark. Samples were analysed on a Guava EasyCyte™ cytometer. Propidium iodide was measured by excitation at 532 nm and collecting emission at 695/50 nm. Data points were collected in triplicate and analysis was performed with InCyte software included in GuavaSoft 3.2 (Millipore).

Both assays confirmed that the CPP alone had low toxicity at the concentrations that were employed in protein transport experiments (1 to 5 $\mu$M). However, viability studies in the presence of CPP at a fixed non-toxic concentration (5 $\mu$M) and increasing amounts of protein (ConA), showed a clear dose-response toxicity effect that was much higher than that for the peptide and the protein alone (Figures 7B, 8B, 7D and 8D). This observation constitutes further proof for the protein delivery, as ConA receptor-mediated delivery has been applied in cytotoxic cancer targeting.

Example 4.3. Study of endosomal escape by confocal microscopy

HeLa cells were incubated with 3 $\mu$M of TmP(Man)$_2$ and TmP(Glu)$_2$ (the TAMRA labelled peptides bearing two mannoses or two glucoses, respectively) diluted in HKR buffer for 30 min. Cells were washed and DMEM with 10 % FBS and 1 % antibiotics was added. Cells were imaged at 30 min since the beginning of the incubation and at 60 min (Fig. 12A) in a confocal microscope. Bright field images (top panels) and fluorescence images of the peptide (bottom panels) were acquired.

TmP(Glu)$_2$ provided a similar endosomal escape, but was less toxic (see results in example 4.2.1, second paragraph) and Fig. 12b.

Example 4.4. Study of endosomal escape by glucocorticoid-induced translocation assay

The ability of the peptide to reach the cytosol was determined with a glucocorticoid induced GFP translocation assay (J. M. Holub, J. R. Larochelle, J. S. Appelbaum, A. Schepartz, Biochemistry 2013, 52, 9036-46.). In this assay, a GFP protein fused to the steroid binding domain of the glucocorticoid receptor (GR) accumulates in the nucleus of the cell in response to dexamethasone binding, so the translocation ratio (the ratio between the nuclear and cytoplasmic fluorescence) can be used as an indicator of the presence of dexamethasone-labelled peptides in the cytosol (Figure 12C). HeLa cells grown in four chamber glass bottom dishes were transfected with the plasmid pK7-GR-GFP (K. L. Carey, S. A. Richards, K. M. Lounsbury, I. G. Macara, J. Cell Biol. 1996, 133, 985-96) using Lipofectamine 2000 and, 24 h post-transfection, cells were washed with HKR and incubated for 30 min with Hoechst 33342 (1 $\mu$M). Cells were then incubated for 1 h with DexP(Man)$_2$ (4 $\mu$M), in the presence or absence of unlabelled ConA (30 nM), with dexamethasone (1 $\mu$M) (positive control) or just with HKR (negative control) and immediately imaged. Twenty to thirty images of each sample were acquired with an Andor Zyla 4.2 digital camera mounted on a Nikon Eclipse Ti-E microscope at 60x magnification and the translocation ratio (the ratio of the median intensities of GFP in the nucleus and in the 2 $\mu$m wide surrounding region) was calculated with CellProfiler as follows: nuclei were identified as Hoechst stained objects using the three-class thresholding Otsu method and the cytoplasmic region was defined as the 2 $\mu$m surrounding area. To ensure a better separation of the cytoplasmic and nuclear region, nuclei were shrunk 0.5 $\mu$m before measuring object intensity. Cells falling below the 20% of the maximum intensity of the image were considered untransfected and discarded for the analysis. A total of 40 to 80 cells were analyzed for each sample.

Example 5: Binding affinity

Fluorescence anisotropy was used to study the binding of a CPP bearing two mannose ligands with its model protein target (ConA). Incubation of the fluorescently-labelled CPP with increasing amounts of native ConA resulted in a gradual increase of the anisotropy, which indicates the formation of a higher molecular weight complex with the protein host. The peptide TmP(Man)$_2$ showed the maximum net anisotropy value and the best equilibrium dissociation constant (Kd = 14 $\pm$ 1 $\mu$M).

The role of the mannose for protein binding was confirmed by removal of the glycans. The CPP with only one mannose residue, TmP(Alloc)(Man), still showed affinity enhancement (Kd = 27 $\pm$ 4 $\mu$M), and the acetone capped CPP (TmP(Acetone)$_2$) showed a marked drop in the binding affinity (Kd = 151 $\pm$ 7 $\mu$M).

**CLAUSES**

**CLAUSE 1:** A cell penetrating peptide comprising a scaffold of at least two hydrophobic blocks and at least two cationic blocks, each cationic block alternating with one hydrophobic block;

wherein the total number of amino acids is comprised between 5 and 40, and
wherein

each of said hydrophobic blocks individually considered consist of one or more hydrophobic amino acids, wherein at least one of said hydrophobic amino acids is selected from the group consisting of leucine, alanine and isoleucine; wherein said at least two hydrophobic blocks comprise at least a leucine amino acid; and

each of said cationic blocks individually considered comprise at least one cationic amino acid having a side chain comprising a nitrogen atom, preferably one which is protonated under physiological conditions.

**CLAUSE 2:** The cell penetrating peptide according to clause 1, wherein said cationic amino acids are each individually selected from the group consisting of lysine, arginine and histidine.

**CLAUSE 3:** The cell penetrating peptide according to any of the previous clauses, wherein at least two cationic blocks comprise at least one arginine amino acid.

**CLAUSE 4:** The cell penetrating peptide according to any of the previous clauses, wherein any of the hydrophobic amino acids does not contain a functional group capable of forming a negative or positive charge under physiological conditions.

**CLAUSE 5:** The cell penetrating peptide according to any of the previous clauses, wherein at least one hydrophobic amino acid of each hydrophobic block is selected from the group consisting of leucine, alanine and isoleucine; and at least one hydrophobic block further comprises at least one hydrophobic amino acid selected from the group consisting of valine, proline, methionine, phenylalanine and tryptophan.

**CLAUSE 6:** The cell penetrating peptide according to any of the previous clauses, wherein each hydrophobic block consists of one or more amino acids selected from the group consisting of alanine, leucine and isoleucine.

**CLAUSE 7:** The cell penetrating peptide according to any of the previous clauses, comprising a reporting moiety.

**CLAUSE 8:** The cell penetrating peptide according to any of the previous clauses, wherein at least two of the hydrophobic blocks each comprise one hydrophobic amino acid selected from leucine, isoleucine, valine, proline, methionine, phenylalanine and tryptophan.

**CLAUSE 9:** The cell penetrating peptide according to clause 8, wherein at least one of the cationic blocks comprises two cationic amino acids selected from lysine, arginine and histidine, preferably two arginine aminoacids.

**CLAUSE 10:** The cell penetrating peptide according to any of the previous clauses, comprising at least one ligand attached to the peptide.

**CLAUSE 11:** The cell penetrating peptide according to clause 10, wherein the ligand is attached to the scaffold through a linker comprising an oxime or a hydrazone group.

**CLAUSE 12:** The cell penetrating peptide according to clause 10, wherein said ligand comprises a monoscharide or an oligosaccharide.

**CLAUSE 13:** The cell penetrating peptide according to clause 12, wherein said monosaccharide or oligosaccharide comprises at least one molecule of glucose or at least one molecule of mannose.

**CLAUSE 14:** The cell penetrating peptide according to any of clauses 12 or 13, wherein said monosaccharide or oligosaccharide comprises at least one molecule of glucose.

**CLAUSE 15:** The cell penetrating peptide according to any of clauses 10 to 14, wherein said ligand is associated to a molecule of biological interest.

**CLAUSE 16:** The cell penetrating peptide according to clause 15, wherein said molecule of biological interest is selected from the group consisting of proteins, polymers and mixtures thereof, preferably a protein, for example an antibody or a lectin.

**CLAUSE 17:** The cell penetrating peptide according to any of the previous clauses, wherein the scaffold comprises a total of 15 to 40 aminoacids, preferably 16 to 25, preferably 16 to 21.

**CLAUSE 18:** The cell penetrating peptide according to clause 17, comprising at least one monoscharide or oligosaccharide.

**CLAUSE 19:** The cell penetrating peptide according to clause 18, comprising at least one molecule of glucose or at least one molecule of mannose.

**CLAUSE 20:** The cell penetrating peptide according to clause 19, wherein said monosaccharide or oligosaccharide comprises at least one molecule of glucose.

**CLAUSE 21:** The cell penetrating peptide according to any of the previous clauses, preferably according to any of clauses 1 to 9, wherein the percentage of helicity in liposomes thereof is greater than 10%, as measured at 40°C in Milli-Q water in egg yolk phosphatidylcholine liposomes.

**CLAUSE 22:** The cell penetrating peptide according to clause 21, wherein the percentage of helicity in liposomes thereof is greater than 12%, preferably greater than 15%, preferably greater than 20%, 22%, 25%, 26%, 27%, 28%, 29%, 30%, 35%, 40% or 45%.

**CLAUSE 23:** The cell penetrating peptide according to any of clauses 21 and 22, wherein the percentage of helicity in liposomes thereof is comprised between 10% and 60%, preferably between 13% and 55%, between 14% and 52%, or between 15% and 50%.

**CLAUSE 25:** The cell penetrating peptide according to any of clauses 1 to 23, wherein the percentage of helicity in water is comprised between 5% and 50%, preferably between 10% and 40%, between 11% and 20%, between 12% and 20%.

**CLAUSE 26:** The cell penetrating peptide according to any of the clauses 21 to 26, wherein the percentage of helicity in liposomes as measured at 40°C in Milli-Q water in egg yolk phosphatidylcholine liposomes is greater than the percentage of helicity in water at 40°C.

**CLAUSE 27:** The cell penetrating peptide according to clause 26, wherein the difference in the percentage of helicity in liposomes as measured at 40°C in Milli-Q water in egg yolk phosphatidylcholine liposomes and the percentage of helicity in water at 40°C is at least 10, preferably at least 15, more preferably at least 20.

**CLAUSE 28:** The cell penetrating peptide according to any of the clauses 17 to 27, wherein at least a first cationic amino acid of a first cationic block is substantially aligned in the direction perpendicular to the theoretical axis of the helix with at least a second cationic amino acid of a second cationic block.

**CLAUSE 29:** The cell penetrating peptide according to clause 28, wherein each cationic block comprises at least one arginine which is substantially aligned in direction perpendicular to the theoretical axis of the helix.

**CLAUSE 30:** The cell penetrating peptide according to any of the clauses 17 to 29, wherein at least two of the hydrophobic blocks each comprise at least one hydrophobic amino acid selected from the group consisting of leucine and isoleucine, and wherein said leucine or isoleucine amino acids are substantially radially distributed around the direction of the theoretical axis of the helix.

**CLAUSE 31:** The cell penetrating peptide according to any of the clauses 28 to 30, wherein the cell penetrating peptide comprises a first half and a second half separated by a plane containing the theoretical axis of the helix formed by the scaffold, wherein the first half comprises at least two hydrophobic amino acids selected from the group consisting of leucine and isoleucine which are substantially radially distributed around the direction of the theoretical axis of the helix, and the second half comprises at least two cationic amino acids substantially aligned in the perpendicular direction of the theoretical axis of the helix.

**CLAUSE 31 (bis):** The cell penetrating peptide according to any of the clauses 28 to 30, wherein the cell penetrating peptide comprises a first half and a second half separated by a plane containing the theoretical axis of the helix formed by the scaffold, wherein the first half comprises at least two hydrophobic amino acids selected from the group consisting of leucine and isoleucine, and the second half comprises at least two cationic amino acids.

**CLAUSE 32:** The cell penetrating peptide according to clause 31, wherein the cell penetrating peptide has a percentage of helicity in liposomes greater than 10%, as measured at 40°C in Milli-Q water in egg yolk phosphatidylcholine liposomes, and comprises a first half and a second half separated by a plane containing the theoretical axis of the helix formed by the scaffold, wherein the first half comprises at least two hydrophobic amino acids selected from the group consisting of leucine and isoleucine substantially radially distributed around the direction of the theoretical axis of the helix, and the second half comprises at least two cationic amino acids substantially aligned in the perpendicular direction of the theoretical axis of the helix.

**CLAUSE 33:** The cell penetrating peptide according to any of the clauses 31 or 32, wherein the cell penetrating peptide has a percentage of helicity in liposomes greater than 10%, as measured at 40°C in Milli-Q water in egg yolk phosphatidylcholine liposomes, and comprises a first half and a second half when separated by a plane containing the theoretical axis of the helix formed by the scaffold, wherein the first half comprises at least two hydrophobic amino acids selected from the group consisting of leucine and isoleucine substantially radially distributed around the direction of the theoretical axis of the helix, each of said leucine and isoleucine pertaining to different hydrophobic blocks, and the second half comprises at least two cationic amino acids substantially aligned in the perpendicular direction of the theoretical axis of the helix, each of said at least cationic amino acids pertaining to different cationic blocks.

**CLAUSE 34:** The cell penetrating peptide according to any of the clauses 17 to 33, wherein at least one of the hydrophobic blocks comprises at least two alanine amino acids.

**CLAUSE 35:** The cell penetrating peptide according to any of the clauses 17 to 34, wherein at least two of the hydrophobic blocks comprise at least two alanine amino acids.

**CLAUSE 36:** A complex comprising

A) a cell penetrating peptide comprising a scaffold of at least two hydrophobic blocks and at least two cationic blocks, each cationic block alternating with one hydrophobic block;
wherein the total number of amino acids is comprised between 5 and 40, and
wherein

each of said hydrophobic blocks individually considered consist of one or more hydrophobic amino acids, wherein at least one of said hydrophobic amino acids is selected from the group consisting of leucine, alanine and isoleucine; wherein said at least two hydrophobic blocks comprise at least a leucine amino acid; and

each of said cationic blocks individually considered comprise at least one cationic amino acid having a side chain comprising a nitrogen atom, preferably one which is protonated under physiological conditions;

B) at least one ligand attached to the cell penetrating peptide; and

C) at least one molecule of biological interest associated to the ligand.

**CLAUSE 36 (bis):** A complex comprising

A) a cell penetrating peptide as defined in any of clauses 1 to 16;

B) at least one ligand attached to the cell penetrating peptide; and

C) at least one molecule of biological interest associated to the ligand.

**CLAUSE 37:** A composition comprising the cell penetrating peptide defined in any of clauses 1 to 7 or 17 to 34 and a molecule of biological interest.
**CLAUSE 38:** A method for preparing the CPP of the invention comprising the reaction between the peptide defined in clause 1 and a ligand.
**CLAUSE 39:** A pharmaceutical composition comprising the cell penetrating peptide as defined in clauses 1 to 35 or a complex as defined in clause 36 or a composition as defined in clause 37.
**CLAUSE 40:** A cell penetrating peptide as defined in clauses 1 to 35 or a complex as defined in clause 36 or a composition as defined in clause 37 for use as a medicament.
**CLAUSE 41:** A cell penetrating peptide as defined in clauses 1 to 35 or a complex as defined in clause 36 or a composition as defined in clause 37 for use in transporting of a molecule of biological interest.
**CLAUSE 42:** A cell penetrating peptide as defined in clauses 1 to 35 or a complex as defined in clause 36 or a composition as defined in clause 37 for use in the treatment of cancer.

**Claims**

1. A cell penetrating peptide comprising a scaffold of at least two hydrophobic blocks and at least two cationic blocks, each cationic block alternating with one hydrophobic block;
   wherein the total number of amino acids is comprised between 5 and 40, and
   wherein

   each of said hydrophobic blocks individually considered consist of one or more hydrophobic amino acids, wherein at least one of said hydrophobic amino acids is selected from the group consisting of leucine, alanine and isoleucine; wherein said at least two hydrophobic blocks comprise at least a leucine amino acid; and
   each of said cationic blocks individually considered comprise at least one cationic amino acid having a side chain comprising a nitrogen atom, preferably one which is protonated under physiological conditions.

2. The cell penetrating peptide according to claim 1, comprising at least one ligand attached to the peptide.

3. The cell penetrating peptide according to claim 2, wherein said ligand comprises a monosacharide or an oligosaccharide.

4. The cell penetrating peptide according to any of claims 2 or 3, wherein said ligand is associated to a molecule of biological interest.

5. The cell penetrating peptide according to any of the previous claims, wherein each hydrophobic block consists of one or more amino acids selected from the group consisting of alanine, leucine and isoleucine.

6. The cell penetrating peptide according to any of claims 1 or 5, wherein the percentage of helicity in liposomes thereof is greater than 10%, as measured at 40°C in Milli-Q water in egg yolk phosphatidylcholine liposomes.

7. The cell penetrating peptide according to claim 6, wherein the percentage of helicity in liposomes as measured at 40°C in Milli-Q water in egg yolk phosphatidylcholine liposomes is greater than the percentage of helicity in water at 40°C.

8. The cell penetrating peptide according to any of claims 6 or 7, wherein at least two of the hydrophobic blocks each comprise at least one hydrophobic amino acid selected from the group consisting of leucine and isoleucine, and wherein said leucine or isoleucine amino acids are substantially radially distributed around the direction of the theoretical axis of the helix.

9. The cell penetrating peptide according to any of the claims 6 to 8, wherein the cell penetrating peptide comprises a first half and a second half separated by a plane containing the theoretical axis of the helix formed by the scaffold, wherein the first half comprises at least two hydrophobic amino acids selected from the group consisting of leucine and isoleucine which are substantially radially distributed around the direction of the theoretical axis of the helix, and the second half comprises at least two cationic amino acids substantially aligned in the perpendicular direction of the theoretical axis of the helix.

10. The cell penetrating peptide according to any of the claims 6 to 9, wherein the cell penetrating peptide has a percentage of helicity in liposomes greater than 10%, as measured at 40°C in Milli-Q water in egg yolk phosphatidylcholine liposomes, and comprises a first half and a second half when separated by a plane containing the theoretical axis of the helix formed by the scaffold, wherein the first half comprises at least two hydrophobic amino acids selected from the group consisting of leucine and isoleucine substantially radially distributed around the direction of the theoretical axis of the helix, each of said leucine and isoleucine pertaining to different hydrophobic blocks, and the second half comprises at least two cationic amino acids substantially aligned in the perpendicular direction of the theoretical axis of the helix, each of said at least cationic amino acids pertaining to different cationic blocks.

11. A complex comprising

   A) a cell penetrating peptide as defined in any of claims 1 to 5;
   B) at least one ligand attached to the cell penetrating peptide; and
   C) at least one molecule of biological interest associated to the ligand.

12. A composition comprising the cell penetrating peptide defined in any of claims 6 to 10 and a molecule of biological interest.

13. A pharmaceutical composition comprising the cell penetrating peptide as defined in claims 1 to 5 or a complex as defined in claim 11 or a composition as defined in claim 12.

14. A cell penetrating peptide as defined in claims 1 to 5 or a complex as defined in claim 11 or a composition as defined in claim 12 for use as a medicament.

15. A cell penetrating peptide as defined in claims 1 to 5 or a complex as defined in claim 11 or a composition as defined in claim 12 for use in the treatment of cancer.

1) 20% piperidine in DMF
2) 4 equiv L-Fmoc-Arg(pbf)-OH
   4 equiv HBTU in DMF
   4 equiv 0.195 M DIEA in DMF } 16 cycles

FmocHN—●

**SPPS elongation (1, 2, 1, 2...)**

FmocHN——LRALAALARAAAAAAR—●

1) 20% piperidine in DMF
2) 1 equiv TAMRA
   1 equiv HATU
   1 equiv DIEA

**Fluorophore capping**

1) 20% piperidine in DMF
2) Ac$_2$O/2,6-Lutidine (1:1)

**Acylation capping**

AcHN—LRALAALARAAAAAAR—●

TAMRA—HN—LRALAALARAAAAAAR—●

TFA/CH$_2$Cl$_2$/H$_2$O/TIS

**Resin cleavage and protecting groups removal**

AcHN—LRALAALARAAAAAAR—CONH$_2$       TAMRA—HN—LRALAALARAAAAAAR—CONH$_2$

Figure 1

Figure 2

Figure 3

Figure 4

Figure 5

Figure 6

Figure 7

Figure 8

Figure 9

EP 3 556 767 A1

Figure 10

Figure 11

Figure 12

**Europäisches Patentamt**
**European Patent Office**
**Office européen des brevets**

## PARTIAL EUROPEAN SEARCH REPORT

under Rule 62a and/or 63 of the European Patent Convention.
This report shall be considered, for the purposes of
subsequent proceedings, as the European search report

Application Number

EP 18 38 2261

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2014/001229 A2 (HOFFMANN-LA ROCHE) 3 January 2014 (2014-01-03) * the whole document * | 1-15 | INV. C07K7/08 |
| X | WO 2010/099510 A2 (TULANE) 2 September 2010 (2010-09-02) * the whole document * | 1-15 | |

**TECHNICAL FIELDS SEARCHED (IPC)**

C07K

### INCOMPLETE SEARCH

The Search Division considers that the present application, or one or more of its claims, does/do not comply with the EPC so that only a partial search (R.62a, 63) has been carried out.

Claims searched completely :

Claims searched incompletely :

Claims not searched :

Reason for the limitation of the search:

see sheet C

| Place of search | Date of completion of the search | Examiner |
|---|---|---|
| The Hague | 15 February 2019 | Masturzo, Pietro |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

&  : member of the same patent family, corresponding document

EPO FORM 1503 03.82 (P04E07)

Europäisches
Patentamt

European
Patent Office

Office européen
des brevets

**INCOMPLETE SEARCH
SHEET C**

Application Number

EP 18 38 2261

Claim(s) completely searchable:
-

Claim(s) searched incompletely:
1-15

Reason for the limitation of the search:

The present application comprises 15 claims, referring to cell-penetrating peptides and their derivatives and applications. These peptides comprise between 5 and 40 residues, where hydrophobic and cationic blocks alternate. However, no fixed structure is given and a non-limitative indication of the nature of a few residues is provided only for the hydrophobic blocks, whose length (as well as those of the cationic block) is not defined. Moreover the applicant should consider that In this case the main claim 1 figures as a reach-through claim, which, in the absence of more than a handful of compounds really prepared and tested, takes the character of a result to be achieved. This makes a complete search impossible, especially because the skilled artisan should determine a cell-penetrating ability for all peptides falling under the huge definition of claim 1. The present claims as filed conflict thus prima facie with Art. 56 (in the light of T939/92), 83 and 84 EPC to such an extent, that no search is deemed possible at present. The applicant has submitted an attempt to clarification consisting of four sets of claims. Those new claims cannot be filed at this stage; however their subject matter will be considered as an indication of the subject matter the applicant would like to be searched and examined. The subject matter on which a search and the examination were carried out correspond therefore to the subject matter of the third auxiliary request as filed by the applicant. The three other requests are considere to comprise unsearchable matter and therefore they fall under the above objections, that is, Artt. 56, 83 and 84 EPC.

EP 3 556 767 A1

**ANNEX TO THE EUROPEAN SEARCH REPORT
ON EUROPEAN PATENT APPLICATION NO.**

EP 18 38 2261

This annex lists the patent family members relating to the patent documents cited in the above-mentioned European search report.
The members are as contained in the European Patent Office EDP file on
The European Patent Office is in no way liable for these particulars which are merely given for the purpose of information.

15-02-2019

| Patent document cited in search report | | | Publication date | Patent family member(s) | | | Publication date |
|---|---|---|---|---|---|---|---|
| WO 2014001229 | A2 | | 03-01-2014 | BR | 112014027239 | A2 | 18-07-2017 |
| | | | | CA | 2869283 | A1 | 03-01-2014 |
| | | | | CN | 104428310 | A | 18-03-2015 |
| | | | | EP | 2864348 | A2 | 29-04-2015 |
| | | | | HK | 1205749 | A1 | 24-12-2015 |
| | | | | JP | 2015522264 | A | 06-08-2015 |
| | | | | KR | 20150032265 | A | 25-03-2015 |
| | | | | RU | 2015102027 | A | 10-08-2016 |
| | | | | US | 2015183827 | A1 | 02-07-2015 |
| | | | | US | 2018094030 | A1 | 05-04-2018 |
| | | | | WO | 2014001229 | A2 | 03-01-2014 |
| WO 2010099510 | A2 | | 02-09-2010 | US | 2012190630 | A1 | 26-07-2012 |
| | | | | WO | 2010099510 | A2 | 02-09-2010 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **G. L. SIMPSON ; A. H. GORDON ; D. M. LINDSAY ; N. PROMSAWAN ; M. P CRUMP ; K. MULHOLLAND ; B. R. HAYTER ; T. GALLAGHER.** *J. Am. Chem. Soc.,* 2006, vol. 128, 10638-10639
- **H. LIS ; N. SHARON.** *Chem. Rev.,* 1998, vol. 98, 637-674
- **N. YAMAZAKI ; S. KOJIMA ; N. V. BOVIN ; S. ANDRE ; S. GABIUS ; H. J. GABIUS.** *Adv. Drug. Deliv. Rev.,* 2000, vol. 43, 225-244
- **A. AMIN ; M. L. OO ; T. SENGA ; N. SUZUKI ; G. S. FENG ; M. HAMAGUCHI.** *Cancer Res.,* 2003, vol. 63, 6334-6339
- **L. DUTOT ; P. LÉCORCHÉ ; F. BURLINA ; R. MARQUANT ; V. POINT ; S. SAGAN ; G. CHASSAING ; J.-M. MALLET ; S. LAVIELLE.** *J. Chem. Biol.,* 2010, vol. 3, 51-65
- **P.M. FISCHER.** *Med.Res.Rev.,* 2007, vol. 27, 755-795
- **D. KALAFATOVIC ; E. GIRALT.** *Molecules,* 2017, vol. 22
- **J. M. HOLUB ; J. R. LAROCHELLE ; J. S. APPELBAUM ; A. SCHEPARTZ.** *Biochemistry,* 2013, vol. 52, 9036-46
- **K. L. CAREY ; S. A. RICHARDS ; K. M. LOUNSBURY ; I. G. MACARA.** *J. Cell Biol.,* 1996, vol. 133, 985-96